(19) Europäisches Patentamt
European Patent Office
Office européen des brevets

(11) EP 4 635 947 A2

(12) EUROPEAN PATENT APPLICATION
published in accordance with Art. 153(4) EPC

(43) Date of publication:
**22.10.2025 Bulletin 2025/43**

(21) Application number: **23902813.7**

(22) Date of filing: **15.12.2023**

(51) International Patent Classification (IPC):
***C07D 309/10*** (2006.01) ***A61P 3/10*** (2006.01)
***A61P 5/50*** (2006.01) ***A61P 3/06*** (2006.01)
***A61P 3/04*** (2006.01) ***A61P 9/10*** (2006.01)
***A61P 9/00*** (2006.01) ***A61P 9/04*** (2006.01)
***A61P 9/12*** (2006.01) ***A61P 13/12*** (2006.01)
***A61K 31/351*** (2006.01) ***A61K 31/7048*** (2006.01)

(52) Cooperative Patent Classification (CPC):
**A61K 31/351; A61P 3/04; A61P 3/06; A61P 3/10; A61P 5/50; A61P 9/00; A61P 9/04; A61P 9/10; A61P 9/12; A61P 13/12; A61P 25/00; A61P 27/02; C07D 309/10**

(86) International application number:
**PCT/CN2023/139009**

(87) International publication number:
**WO 2024/125620 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2022 CN 202211622377**

(71) Applicant: **Sunshine Lake Pharma Co., Ltd.**
**Dongguan, Guangdong 523000 (CN)**

(72) Inventors:
- **CHEN, Liang**
  **Dongguan, Guangdong 523871 (CN)**
- **GU, Zheng**
  **Dongguan, Guangdong 523871 (CN)**
- **WU, Wuyong**
  **Dongguan, Guangdong 523871 (CN)**
- **SUN, Tao**
  **Dongguan, Guangdong 523871 (CN)**
- **ZHANG, Yingjun**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) CRYSTAL FORM OF GLUCOPYRANOSYL DERIVATIVE AND USE THEREOF

(57) The present invention relates to a crystal form of a glucopyranosyl derivative and the use thereof. Specifically, the present invention relates to a crystal form of *N*-(2-dimethylaminoethyl)-1-[4-[4-[[5-[(2*S*,3*R*,4*S*,5*S*,6*R*)-6-ethyl-3,4,5-trihydroxy-tetrahydropyran-2-yl]-2-methyl-phenyl]methyl]phenyl]butyrylamino]cyclohexylformamide and a pharmaceutical composition comprising the crystal form, and further relates to the use of the crystal form and the pharmaceutical composition in the preparation of a sodium-dependent glucose transporter (SGLTs) inhibitor.

EP 4 635 947 A2

## Description

### FIELD OF THE INVENTION

The present invention relates to a crystal form of a glucopyranosyl derivative and a pharmaceutical composition comprising the crystal form of the present invention, as well as use thereof in the preparation of sodium-dependent glucose transporters (SGLTs) inhibitors.

### BACKGROUND ART

SGLTs inhibitors can be used to treat diabetes-related complications, such as retinopathy, neuropathy, kidney disease, insulin resistance caused by glucose metabolic disorder, hyperinsulinemia, hyperlipidemia, obesity, and so on. Meanwhile, SGLTs inhibitors can also be used in combination with current treatment regimens, such as sulphonamides, thiazolidinedione, metformin, and insulin, etc, which can reduce the dose without impacting on the effectiveness of the medicine, and thereby avoid or reduce side effects, and improve patient compliance. Currently, marketed SGLTs inhibitors include canagliflozin and dapagliflozin, etc., which are mainly used for the treatment of type II diabetes and complications thereof.

Patent application WO2021004498A1 discloses a class of glucopyranosyl derivative as SGLTs inhibitors, specifically disclosing N-(2-dimethylaminoethyl)-1-[4-[4-[[5-[(2S,3R,4S,5S,6R)-6-ethyl-3,4,5-trihydroxy-tetrahydropyran-2-yl]-2-methylphenyl]methyl]phenyl]butanoylamino]cyclohexyl formamide (i.e., the compound shown in formula (I) of this application), which has a significant inhibitory effect on SGLTs. The product obtained by the preparation method described in this patent application is amorphous, with poor stability, and does not meet the requirements for storage and formulation.

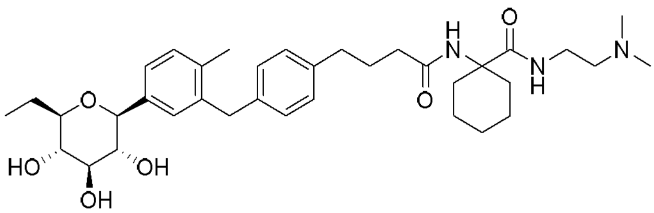

(I)

### SUMMARY

This invention specifically relates to the crystal form of N-(2-dimethylaminoethyl)-1-[4-[4-[[5-[(2*S*,3*R*,4*S*,5*S*,6*R*)-6-ethyl-3,4,5-trihydroxy-tetrahydropyran-2-yl]-2-methylphenyl]methyl]phenyl] butanoylamino] cyclohexyl formamide, specifically including crystal form I and crystal form II. The crystal forms prepared by this invention can be identified and distinguished from other crystal forms using their characteristic X-ray powder diffraction (XRPD) patterns, differential scanning calorimetry (DSC) curves, thermogravimetric analysis (TGA) curves, Raman spectrum, and Fourier-transform infrared (FT-IR) spectrum. The crystal form I described in this invention has appropriate solubility, good stability, and low hygroscopicity, thus has excellent development prospects.

In one aspect, the present invention provides a crystal form of the compound shown in formula (I);

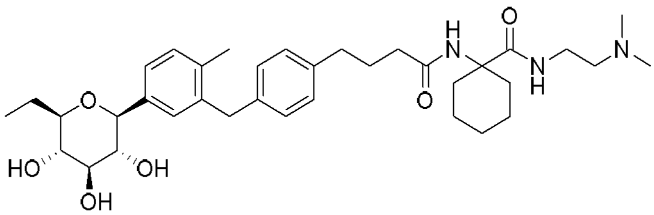

(I).

In some embodiments, the crystal form of the compound shown in formula (I) in the present invention is crystal form I or II.

In some embodiments, the X-ray powder diffraction pattern of crystal form I described in this invention comprises diffraction peaks at the following 2θ angles: 5.02° ± 0.2°, 9.99° ± 0.2°, 12.67° ± 0.2°, 14.96° ± 0.2°, 15.55° ± 0.2°, 20.64° ± 0.2°.

In other embodiments, the X-ray powder diffraction pattern of crystal form I described in this invention comprises

diffraction peaks at the following 20 angles: 5.02° ± 0.2°, 9.06° ± 0.2°, 9.99° ± 0.2°, 12.67° ± 0.2°, 13.47° ± 0.2°, 14.96° ± 0.2°, 15.55° ± 0.2°, 16.16° ± 0.2°, 18.19° ± 0.2°, 20.64° ± 0.2°, 25.02° ± 0.2°.

In other embodiments, the X-ray powder diffraction pattern of crystal form I described in this invention comprises diffraction peaks at the following 20 angles: 5.02° ± 0.2°, 5.72° ± 0.2°, 7.16° ± 0.2°, 9.06° ± 0.2°, 9.99° ± 0.2°, 10.27° ± 0.2°, 11.36° ± 0.2°, 12.67° ± 0.2°, 13.47° ± 0.2°, 14.29° ± 0.2°, 14.96° ± 0.2°, 15.55° ± 0.2°, 16.16° ± 0.2°, 16.84° ± 0.2°, 16.96° ± 0.2°, 17.68° ± 0.2°, 18.19° ± 0.2°, 18.36° ± 0.2°, 18.52° ± 0.2°, 18.94° ± 0.2°, 19.78° ± 0.2°, 20.64° ± 0.2°, 21.10° ± 0.2°, 21.44° ± 0.2°, 21.85° ± 0.2°, 22.22° ± 0.2°, 22.48° ± 0.2°, 23.08° ± 0.2°, 23.69° ± 0.2°, 23.80° ± 0.2°, 25.02° ± 0.2°, 25.43° ± 0.2°, 25.70° ± 0.2°, 26.38° ± 0.2°, 26.74° ± 0.2°, 27.18° ± 0.2°, 27.68° ± 0.2°, 28.62° ± 0.2°, 29.53° ± 0.2°, 30.01° ± 0.2°, 30.52° ± 0.2°, 31.08° ± 0.2°, 31.53° ± 0.2°, 32.01° ± 0.2°, 32.60° ± 0.2°.

In other embodiments, the X-ray powder diffraction pattern of crystal form I of the present invention comprises diffraction peaks at the following 2θ angles: 5.02° ± 0.2°, 5.72° ± 0.2°, 7.16° ± 0.2°, 9.06° ± 0.2°, 9.99° ± 0.2°, 10.27° ± 0.2°, 10.55° ± 0.2°, 11.36° ± 0.2°, 12.67° ± 0.2°, 13.47° ± 0.2°, 14.29° ± 0.2°, 14.96° ± 0.2°, 15.55° ± 0.2°, 16.16° ± 0.2°, 16.84° ± 0.2°, 16.96° ± 0.2°, 17.68° ± 0.2°, 18.19° ± 0.2°, 18.36° ± 0.2°, 18.52° ± 0.2°, 18.94° ± 0.2°, 19.78° ± 0.2°, 20.64° ± 0.2°, 21.10° ± 0.2°, 21.44° ± 0.2°, 21.85° ± 0.2°, 22.22° ± 0.2°, 22.48° ± 0.2°, 23.08° ± 0.2°, 23.69° ± 0.2°, 23.80° ± 0.2°, 25.02° ± 0.2°, 25.43° ± 0.2°, 25.70° ± 0.2°, 26.38° ± 0.2°, 26.74° ± 0.2°, 27.18° ± 0.2°, 27.68° ± 0.2°, 28.62° ± 0.2°, 29.53° ± 0.2°, 30.01° ± 0.2°, 30.52° ± 0.2°, 31.08° ± 0.2°, 31.53° ± 0.2°, 32.01° ± 0.2°, 32.60° ± 0.2°, 33.34° ± 0.2°, 33.92° ± 0.2°, 34.28° ± 0.2°, 34.57° ± 0.2°, 35.31° ± 0.2°, 35.73° ± 0.2°, 36.25° ± 0.2°, 36.90° ± 0.2°, 37.37° ± 0.2°, 37.86° ± 0.2°, 38.43° ± 0.2°, 38.92° ± 0.2°, 39.46° ± 0.2°.

In other embodiments, the X-ray powder diffraction pattern of crystal form I of the invention comprises diffraction peaks at the following 2θ angles: 5.02° ± 0.2°, 5.72° ± 0.2°, 7.16° ± 0.2°, 9.06° ± 0.2°, 9.99° ± 0.2°, 10.27° ± 0.2°, 10.55° ± 0.2°, 11.36° ± 0.2°, 12.67° ± 0.2°, 13.47° ± 0.2°, 14.29° ± 0.2°, 14.96° ± 0.2°, 15.55° ± 0.2°, 16.16° ± 0.2°, 16.84° ± 0.2°, 16.96° ± 0.2°, 17.68° ± 0.2°, 18.19° ± 0.2°, 18.36° ± 0.2°, 18.52° ± 0.2°, 18.94° ± 0.2°, 19.78° ± 0.2°, 20.64° ± 0.2°, 21.10° ± 0.2°, 21.44° ± 0.2°, 21.85° ± 0.2°, 22.22° ± 0.2°, 22.48° ± 0.2°, 23.08° ± 0.2°, 23.69° ± 0.2°, 23.80° ± 0.2°, 25.02° ± 0.2°, 25.43° ± 0.2°, 25.70° ± 0.2°, 26.38° ± 0.2°, 26.74° ± 0.2°, 27.18° ± 0.2°, 27.68° ± 0.2°, 28.62° ± 0.2°, 29.53° ± 0.2°, 30.01° ± 0.2°, 30.52° ± 0.2°, 31.08° ± 0.2°, 31.53° ± 0.2°, 32.01° ± 0.2°, 32.60° ± 0.2°, 33.34° ± 0.2°, 33.92° ± 0.2°, 34.28° ± 0.2°, 34.57° ± 0.2°, 35.31° ± 0.2°, 35.73° ± 0.2°, 36.25° ± 0.2°, 36.90° ± 0.2°, 37.37° ± 0.2°, 37.86° ± 0.2°, 38.43° ± 0.2°, 38.92° ± 0.2°, 39.46° ± 0.2°, 40.00° ± 0.2°, 40.94° ± 0.2°, 41.54° ± 0.2°, 41.92° ± 0.2°, 42.39° ± 0.2°, 42.99° ± 0.2°, 43.48° ± 0.2°, 44.08° ± 0.2°, 44.72° ± 0.2°, 46.41° ± 0.2°, 47.24° ± 0.2°, 48.64° ± 0.2°, 50.41° ± 0.2°, 51.52° ± 0.2°, 53.19° ± 0.2°, 53.99° ± 0.2°, 58.28° ± 0.2°.

In some embodiments, the compound of formula (I) described in the invention is crystal form II.

In some embodiments, the X-ray powder diffraction pattern of crystal form II described in the invention comprises diffraction peaks at the following 20 angles: 4.90° ± 0.2°, 14.77° ± 0.2°, 15.89° ± 0.2°, 16.29° ± 0.2°, 17.89° ± 0.2°, 18.51° ± 0.2°.

In other embodiments, the X-ray powder diffraction pattern of crystal form II described in the invention comprises diffraction peaks at the following 2θ angles: 4.90° ± 0.2°, 9.82° ± 0.2°, 12.46° ± 0.2°, 14.77° ± 0.2°, 15.89° ± 0.2°, 16.29° ± 0.2°, 17.89° ± 0.2°, 18.51° ± 0.2°, 18.92° ± 0.2°, 20.31° ± 0.2°, 20.77° ± 0.2°.

In other embodiments, the X-ray powder diffraction pattern of crystal form II described in the invention comprises diffraction peaks at the following 20 angles: 4.90° ± 0.2°, 5.56° ± 0.2°, 7.00° ± 0.2°, 8.90° ± 0.2°, 9.82° ± 0.2°, 10.04° ± 0.2°, 10.33° ± 0.2°, 11.16° ± 0.2°, 12.46° ± 0.2°, 13.28° ± 0.2°, 14.06° ± 0.2°, 14.77° ± 0.2°, 15.28° ± 0.2°, 15.89° ± 0.2°, 16.29° ± 0.2°, 16.80° ± 0.2°, 16.99° ± 0.2°, 17.89° ± 0.2°, 18.31° ± 0.2°, 18.51° ± 0.2°, 18.92° ± 0.2°, 19.70° ± 0.2°, 20.31° ± 0.2°, 20.77° ± 0.2°, 21.17° ± 0.2°, 21.71° ± 0.2°, 21.91° ± 0.2°, 22.15° ± 0.2°, 22.80° ± 0.2°, 23.08° ± 0.2°, 23.65° ± 0.2°, 24.75° ± 0.2°, 25.07° ± 0.2°, 25.57° ± 0.2°, 25.86° ± 0.2°, 26.39° ± 0.2°, 27.07° ± 0.2°, 27.30° ± 0.2°, 27.59° ± 0.2°, 28.24° ± 0.2°, 29.13° ± 0.2°, 29.77° ± 0.2°, 30.14° ± 0.2°, 30.15° ± 0.2°, 30.47° ± 0.2°, 31.00° ± 0.2°, 31.17° ± 0.2°, 31.38° ± 0.2°, 31.85° ± 0.2°, 32.41° ± 0.2°, 33.00° ± 0.2°, 33.57° ± 0.2°, 34.08° ± 0.2°, 34.93° ± 0.2°, 36.00° ± 0.2°, 36.32° ± 0.2°, 37.60° ± 0.2°, 38.68° ± 0.2°, 39.20° ± 0.2°.

In some embodiments, the crystal form I of the compound shown in formula (I) in the present invention, wherein the crystal form I has the following unit cell parameters: unit cell dimensions: a= 5.81961 Å, b = 17.21305 Å, c = 35.51838 Å, α = 90°, β = 90°, γ = 90°;

Space group: Orthorhombic, $P2_12_12_1$;
Unit cell volume: 3557.99 $Å^3$;
The number of asymmetric units Z in the unit cell is: 4.

In some embodiments, the crystal form I of the compound shown in formula (I) of the present invention, wherein the differential scanning calorimetry diagram of the crystal form I comprises an endothermic peak at 129.31°C ± 3°C.

In some embodiments, the crystal form I of the compound shown in formula (I) of the present invention, wherein the crystal form I has an X-ray powder diffraction pattern substantially as shown in Figure 1.

In some embodiments, the crystal form I of the compound shown in formula (I) of the present invention, wherein

the crystal form I has a differential scanning calorimetry diagram substantially as shown in Figure 2.

In some embodiments, the crystal form I of the compound shown in formula (I) of the present invention, wherein the crystal form I has a thermogravimetric analysis chart substantially as shown in Figure 3.

In some embodiments, the crystal form II of the compound shown in formula (I) in the present invention, wherein the crystal form II has an X-ray powder diffraction pattern substantially as shown in Figure 4.

In some embodiments, the amorphous form of the compound shown in formula (I) in the present invention, wherein the amorphous form has an X-ray powder diffraction pattern substantially as shown in Figure 6.

In some embodiments, the preparation method of crystal form I of the compound shown in formula (I) in the present invention involves the compound shown in formula (I) and L-proline in an organic solvent, heating, stirring, cooling, precipitating crystals, and filtering to obtain a cocrystal of the compound shown in formula (I) and L-proline. Then, a mixed solvent is added, heated, and crystals are precipitated, filtered, and the filter cake is dried to obtain crystal form I of the compound shown in formula (I).

In some embodiments, the preparation method of crystal form I of the compound shown in formula (I) in the present invention comprises the compound shown in formula (I) and L-proline in ethanol, heated to 60°C, stirred, cooled to room temperature, precipitated crystals, filtered, and after drying the filter cake, obtaining a cocrystal of the compound shown in formula (I) and L-proline. Then, a mixed solvent of ethanol and water is added, heated to 40°C, precipitated crystals, stirred, filtered, and after drying the filter cake, obtaining crystal form I of the compound shown in formula (I).

In another embodiment, in the preparation method of crystal form I of the compound shown in formula (I) of the present invention, the organic solvent is selected from methanol, ethanol, isopropanol, or n-propanol, and the mixed solvent is selected from a mixed solvent of methanol and water, ethanol and water, isopropanol and water, or n-propanol and water.

In some embodiments, the preparation method of crystal form I of the compound shown in formula (I) in the present invention, where the co-crystal of L-proline can be any co-crystal formed by the compound shown in formula (I) and L-proline, including but not limited to crystal form B, etc.

In some embodiments, the preparation method of the crystal form I of the compound shown in formula (I) of the present invention, wherein the L-proline co-crystal can be the co-crystal B of the compound L-proline shown in formula (I), and its X-ray powder diffraction pattern comprises diffraction peaks at the following 2θ angles: 4.17° ± 0.2°, 5.50° ± 0.2°, 8.25° ± 0.2°, 10.46° ± 0.2°, 10.88° ± 0.2°, 11.20° ± 0.2°, 11.48° ± 0.2°, 12.30° ± 0.2°, 12.80° ± 0.2°, 13.79° ± 0.2°, 14.95° ± 0.2°, 15.55° ± 0.2°, 16.06° ± 0.2°, 16.53° ± 0.2°, 16.75° ± 0.2°, 17.22° ± 0.2°, 17.63° ± 0.2°, 18.04° ± 0.2°, 18.71° ± 0.2°, 19.00° ± 0.2°, 19.37° ± 0.2°, 19.61° ± 0.2°, 19.87° ± 0.2°, 20.12° ± 0.2°, 20.48° ± 0.2°, 21.58° ± 0.2°, 22.06° ± 0.2°, 22.27° ± 0.2°, 22.62° ± 0.2°, 23.44° ± 0.2°, 23.89° ± 0.2°, 24.48° ± 0.2°, 24.92° ± 0.2°, 25.77° ± 0.2°, 26.19° ± 0.2°, 26.94° ± 0.2°, 30.73° ± 0.2°, 31.26° ± 0.2°, 31.88° ± 0.2°, 35.21° ± 0.2°, 39.84° ± 0.2°, 41.06° ± 0.2°, and 42.33° ± 0.2°.

In some embodiments, the co-crystal of the compound shown in formula (I) of the present invention and L-proline is a co-crystal form B, wherein the cocrystal crystal form B has an X-ray powder diffraction pattern substantially as shown in Figure 10.

In some embodiments, the pharmaceutical composition of the present invention, which comprises any crystal form of the present invention, further comprises a pharmaceutically acceptable carrier, excipient, adjuvant, vehicle, or a combination thereof.

In some embodiments, the pharmaceutical composition of the present invention further comprises one or more additional therapeutic agents, wherein the additional therapeutic agents are selected from antidiabetic drugs, antihyperglycemic drugs, anti-obesity drugs, antihypertensive drugs, appetite suppressants, lipid-lowering drugs, or combinations thereof.

In some embodiments, the antidiabetic drugs and antihyperglycemic drugs of the present invention are independently selected from SGLT2 inhibitors, biguanides, sulfonylureas, glucosidase inhibitors, PPAR agonists, αP2 inhibitors, PPARα/γ dual activators, dipeptidyl peptidase IV inhibitors, glinides, insulin, glucagon-like peptide-1 inhibitors, PTP1B inhibitors, glycogen phosphorylase inhibitors, glucose-6-phosphatase inhibitors, or combinations thereof. The anti-obesity drugs are selected from central anti-obesity drugs, MCH receptor antagonists, neuropeptide Y receptor antagonists, cannabinoid receptor antagonists, brain-gut peptide antagonists, lipase inhibitors, β3 agonists, 11β-HSD1 inhibitors, DGAT-1 inhibitors, peptide appetite suppressants, cholecystokinin agonists, appetite suppressants, or combinations thereof. The lipid-lowering drugs are selected from MTP inhibitors, HMG-CoA reductase inhibitors, squalene synthase inhibitors, betaine lipid-lowering drugs, ACAT inhibitors, lipoxygenase inhibitors, cholesterol absorption inhibitors, ileal sodium/bile acid co-transporter inhibitors, LDL receptor activity upregulators, niacin lipid-lowering drugs, bile acid chelates, or combinations thereof. The lipid-lowering drugs are selected from pravastatin, simvastatin, atorvastatin, fluvastatin, cerivastatin, pitavastatin, rosuvastatin, or combinations thereof.

In one aspect, the present invention relates to the crystal form I of a compound shown in formula (I) as described in the present invention, or pharmaceutical composition thereof for use in the preparation of a medicament, wherein the medicament is used to inhibit SGLT1.

In other aspect, the present invention relates to the crystal form I of a compound shown in formula (I) as described in the present invention, or pharmaceutical composition thereof for use in the preparation of a medicament, wherein the medicament is used for preventing or treating diseases, alleviating symptoms of the disease, or delaying the progression or onset of the disease.

In some embodiments, the disease described in this invention is diabetes, diabetic complications, insulin resistance, hyperglycemia, hyperinsulinemia, hyperlipidemia, obesity, syndrome X, atherosclerosis, cardiovascular diseases, congestive heart failure, hypomagnesemia, hyponatremia, renal failure, disorders related to hemoconcentration, constipation, or hypertension; wherein the diabetic complications are diabetic retinopathy, diabetic neuropathy, or diabetic nephropathy; and the hyperlipidemia is hypertriglyceridemia.

The foregoing merely summarizes certain aspects disclosed herein and is not intended to be limiting in nature. These aspects and other aspects and embodiments are described more fully below.

## DETAILED DESCRIPTION OF THE INVENTION

The invention relates to the crystal form of the compound N-(2-dimethylaminoethyl)-1-[4-[4-[[5-[(2S,3R,4S,SS,6R)-6-ethyl-3,4,5-trihydroxy-tetrahydropyran-2-yl]-2-methylphenyl]methyl]phenyl] butanoylamino] cyclohexyl formamide, and the preparation method of the crystal form. It also involves pharmaceutical compositions containing the crystal form and the application of the crystal form and pharmaceutical compositions in medicine.

Of particular note is that all similar substitutions and modifications to the skilled person is obvious, and they are deemed to be included in the present invention. In this invention, the crystal form of the compound shown in formula (I) may contain a certain amount of moisture or solvent. Crystal forms containing a certain amount of moisture or other solvents are considered to be within the scope of this invention as long as they possess any characteristic of crystal form I or crystal form II described in this invention. After reading the following detailed description, those skilled in the art can more easily understand the features and advantages of this invention. It should be understood that, for clarity, certain features of the invention described in the context of separate embodiments above and below may also be combined to form a single embodiment. Conversely, for brevity, different features of the invention described in the context of a single embodiment may also be combined to form sub-combinations thereof.

## DEFINITIONS AND GENERAL TERMINOLOGY

Unless otherwise specified, the terms used in the specification and claims of this invention have the following definitions.

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application (including but not limited to defined terms, term usage, described techniques, or the like), the present application shall prevail.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one (i.e. at least one) of the grammatical objects of the article. By way of example, "a component" means one or more components, and thus, possibly, more than one component is contemplated and may be employed or used in an

implementation of the described embodiments.

The term "cocrystal" or "co-crystal" used in this invention refers to a specific crystal structure formed by the active component and a suitable cocrystal former (also known as a ligand) through molecular recognition, without disrupting the chemical bonds of the active component itself. This is achieved through intermolecular forces such as hydrogen bonds, halogen bonds, π-stacking interactions, and van der Waals forces, which are non-covalent bonds. There is a fixed stoichiometric ratio between the components in a cocrystal. A cocrystal is a multi-component crystal, which includes binary cocrystals formed between two neutral solids, as well as multicomponent cocrystals formed between a neutral solid and a salt or solvate. For pharmaceutical active ingredients, the crystalline form can affect many physicochemical properties, which directly impact the ability to process and/or prepare the drug and its corresponding final dosage form. For example, cocrystals can improve the solubility, hydroscopicity, stability of the bulk drug, and its manufacturability (such as compressibility, fluidity, and filterability), and also affect the drug's stability, dissolution rate, and bioavailability. Cocrystals can influence the quality, safety, and efficacy of the drug.

As used herein, the term "subject" refers to an animal. Typically, the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

The terms "subject" and "patient" as used in this invention are used interchangeably. The terms "subject" and "patient" refer to animals (e.g., birds such as chickens, quails, or turkeys, or mammals), particularly "mammals" including non-primate animals (e.g., cattle, pigs, horses, sheep, rabbits, guinea pigs, rats, cats, dogs, and mice) and primates (e.g., monkeys, chimpanzees, and humans), more specifically humans. In one embodiment, the subject is a non-human animal, such as livestock (e.g., horses, cattle, pigs, or sheep) or pets (e.g., dogs, cats, guinea pigs, or rabbits). In other embodiments, "patient" refers to a human.

The term "equivalent" number used in this invention is based on the equivalent relationship of the chemical reaction, using the basic raw material in each step as the standard (1 equivalent), and the equivalent amount of other raw materials required.

The term "comprise" or "include" as used in this invention is an open expression, it means comprising the contents disclosed herein, but don't exclude other contents.

In this invention, the crystal form can be considered as characterized by graphical data "depicted" in charts. These data include, for example, X-ray single crystal diffraction patterns, X-ray powder diffraction patterns, Raman spectra, Fourier transform-infrared spectra, DSC curves, and solid-state NMR spectra. Technicians will understand that graphical representations of such data may undergo slight variations (such as relative peak intensity and peak position) due to factors like changes in instrument response and variations in sample concentration and purity, which are known to technicians. Nevertheless, technicians can compare the graphical data in the invention's figures with the graphical data generated for unknown crystal forms and can confirm whether the two sets of graphical data characterize the same crystal form.

"XRD"refers to X-ray Diffraction.

As used herein, the term "amorphous" or "amorphous form" is intended to mean that the substance, component or product in question lacks a characteristic crystal shape or crystal structure, such as when determined by XRPD, is not substantially crystal or the substance, component or product in question, for example, it may not be birefringent or stereoscopic when viewed using a polarized light microscope, or the X-ray powder diffraction pattern may not have sharp peaks. In certain embodiments, a sample comprising an amorphous form of a substance may be substantially free of other amorphous forms and/or crystal forms.

The term "polymorphic" or "polymorphism" as used herein is defined as the possibility of having at least two different crystal arrangements for the same chemical molecule.

Techniques well-known to those skilled in the art can be used to detect, identify, classify, and qualify polymorphic substances. These techniques include, but are not limited to: Differential Scanning Calorimetry (DSC), Thermogravimetric Analysis (TGA), X-ray Powder Diffraction (XRPD), Single Crystal X-ray Diffraction, Vibrational Spectroscopy, Solution Calorimetry, Solid-State Nuclear Magnetic Resonance (SSNMR), Fourier Transform Infrared Spectroscopy (FT-IR spectrum), Raman Spectroscopy, Hot Stage Optical Microscopy, Scanning Electron Microscopy (SEM), Electron Crystallography, as well as quantitative analysis, Particle Size Analysis (PSA), surface area analysis, solubility, and dissolution rate. Polymorphism can be described as the ability of a specific compound to crystallize in different crystal forms while maintaining the same chemical structural formula. The polymorphs of a given substance are chemically equivalent, containing the same atoms bonded in the same manner, but their crystal forms differ, which can affect one or more physical properties, such as dissolution rate, melting point, bulk density, stability, fluidity, and so on.

Unless otherwise indicated, when the present invention refers to a spectrum or data presented in graphical form (e.g., XRPD, FT-IR, Raman and NMR spectra), the term "peak" refers to a peak not caused by background noise, or other special features that can be identified by one of ordinary skill in the art. The term "effective peak" refers to a peak that is at least of intermediate size (e.g., height) of other peaks in the spectrum or data, or at least 1.5, 2, or 2.5 times the intermediate size of other peaks in the spectrum or data.

"Crystal water", also known as "hydration water", is a certain number of water molecules that are combined in the form of neutral water molecules with ions or molecules in the crystal structure, occupying a certain position in the crystal structure. For example, in the crystal of copper (II) sulfate pentahydrate ($CuSO_4 \cdot 5H_2O$), there are 5 crystal water molecules.

"Substantially identical" in the context of X-ray powder diffraction, DSC curves, Raman spectra, and Fourier transform-infrared spectra means that at least 50%, or at least 60%, or at least 70%, or at least 80%, or at least 90%, or at least 95%, or at least 99% of the peaks are displayed in the X-ray powder diffraction pattern, DSC curve, Raman spectrum, and Fourier transform-infrared spectrum, respectively.

As is well known in the field of X-ray powder diffraction (XRPD), for any specified crystal form, the device used, humidity, temperature, orientation of the powder crystals, and other parameters can cause some variability in the appearance, intensity, and position of peaks in the diffraction pattern. For example, see The United States Pharmacopeia #23, National Formulary #18, pages 1843-1844, 1995. In the current situation, the variability of ± 0.2° 2θ in peak position accounts for these possible changes without hindering the clear identification of the indicated crystal form. The identification of the crystal form can be based on any unique differential peaks (measured in ° 2θ) or their combination, typically the more prominent peaks. Therefore, in some embodiments, the crystalline compound of the present invention is characterized by an XRPD pattern having certain peak positions, which exhibits substantially identical features to the XRPD pattern provided in the drawings of the present invention. According to the condition of the instrument used in this test, the diffraction peak has an error tolerance of ± 0.2°. For example, an X-ray powder diffraction pattern "substantially identical" as that provided in Figure 1 of the present invention refers to an XRPD pattern that a person skilled in the art would consider having the same crystal form as the compound in the XRPD pattern of Figure 1. That is, the XRPD pattern of the crystal form may be the same as the XRPD pattern in the figures, or more likely, it may be slightly different. Such an XRPD pattern may not necessarily display every peak presented in the diffraction pattern herein, and/or may show slight variations in the appearance, intensity, or displacement of the peaks due to differences in conditions involved in obtaining the data. A person skilled in the art can determine whether a sample of the crystalline compound has the same or a different crystal form as disclosed in the present invention by comparing their XRPD patterns. Similarly, a person skilled in the art can determine whether the diffraction angles obtained from a given XRPD pattern (expressed in ° 2θ) are in substantially the same positions as the values presented in the present invention. In the context of the present invention, the 2θ values in the X-ray powder diffraction pattern are in degrees (°).

Similarly, as is well known in the field of differential scanning calorimetry (DSC), the melting peak height in a DSC curve depends on many related factors, such as sample preparation and instrument conditions, whereas the peak position is relatively insensitive to experimental details. Therefore, in some embodiments, the crystalline compound of the present invention is characterized by a DSC thermogram having characteristic peak positions and exhibiting properties substantially identical to those of the DSC thermogram provided in the figures herein. According to the instrument conditions used in this test, the melting peak has an error tolerance of ± 3 °C, ± 4 °C, or ± 5 °C. For example, a DSC curve that is "substantially identical" to Figure 2 provided herein refers to a DSC curve that a person skilled in the art would recognize as corresponding to a compound having the same crystalline form as that represented by the DSC curve in Figure 2. That is, while the DSC curve may be identical to the DSC curve shown in the figures, it more likely may exhibit slight variations. The melting peak of such a DSC curve need not exactly match those shown in the DSC curves presented herein and/or may demonstrate minor variations in peak morphology, enthalpy of fusion, or melting temperature arising from differences in experimental conditions during data acquisition.

As is well understood in the field of Raman spectroscopy, the positions and shapes of spectral bands in a Raman spectrum depend on the frequency of scattered light resulting from interactions between sample molecules and incident radiation. Therefore, in some embodiments, the crystalline compound of the present invention is characterized by a Raman spectrum exhibiting characteristic spectral band positions and shapes with properties substantially identical to those of the Raman spectrum provided in the figures herein. According to the condition of the instrument used in this test, the absorption peak has an error tolerance of ± 0.1 $cm^{-1}$.

As is well-known in the field of Fourier transform infrared (FT-IR) spectroscopy, the positions and shapes of absorption bands in an infrared spectrum depend on the energy level transitions of covalent bond vibrations within the sample molecules. Therefore, in some embodiments, the crystalline compound of the present invention is characterized by an FT-IR spectrum exhibiting characteristic absorption band positions and shapes with properties substantially identical to those of the FT- IR spectrum provided in the figures herein. According to Chinese Pharmacopoeia (2015 Edition) General Rule 0402 "Infrared Spectrophotometry" and the experimental instrumentation used herein, the tolerance limits for absorption band positions are ± 5 $cm^{-1}$ near 3000 $cm^{-1}$ and ±2 $cm^{-1}$ near 1000 $cm^{-1}$.

The term "combination" refers to a crystalline form containing its tautomers, wherein the purity of the crystalline form relative to its tautomers is at least 60%, or at least 70%, or at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%; or a crystalline form containing one or more other crystalline forms, wherein the purity of the crystalline form relative to the other crystalline form(s) is at least 60%, or at least 70%, or at least 80%, or at least 85%, or at least 90%, or at

least 93%, or at least 95%, or at least 98%, or at least 99%, or at least 99.5%, or at least 99.6%, or at least 99.7%, or at least 99.8%, or at least 99.9%; or a crystalline form containing other crystalline form(s), wherein the percentage of the other crystalline form(s) by total volume or total weight is less than 20%, or less than 10%, or less than 5%, or less than 3%, or less than 1%, or less than 0.5%, or less than 0.1%, or less than 0.01%.

"Relative strength" means the ratio of the intensity of the other peaks to the intensity of the first strong peak when the intensity of the first strong peak in all the diffraction peaks of the X-ray powder diffraction pattern (XRD) is 100%.

Whenever a number with a value of N is disclosed, any number with a value of N ± 0.01, N ± 0.02, N ± 0.03, N ± 0.05, N ± 0.07, N ± 0.08, N ± 0.1, N ± 0.15, N ± 0.2, N ± 1, N ± 2, N ± 1.5, N ± 3, N ± 4, N ± 5, N ± 6, N ± 7, N ± 8, N ± 9, or N ± 10 is explicitly disclosed, where "±" means plus or minus. Whenever a numerical range disclosing a lower limit RL and an upper limit RU is disclosed, every numerical value within said disclosed range is expressly disclosed.

The crystal form C of the compound of Formula (I) according to the present invention exists in a substantially pure crystalline state.

The term "substantially pure" refers to both chemical purity and polymorphic purity, specifically indicating that one crystalline form is substantially free of one or more other polymorphic forms, that means the purity of the crystalline form is at least 60%, or at least 70%, or at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or crystal form containing other crystal form. The percentage of the other crystals in the total volume or total weight of the crystal form is less than 30%, or less than 20%, or less than 10%, or less than 5%, or less than 3%, or less than 1%, or less than 0.5%, or less than 0.1%, or less than 0.01%.

The purity of the crystal of this invention can be determined by known methods such as X-ray powder diffraction, thermal analysis, etc. The purity of the crystal or mixed crystal in the present invention need not be 100%, and may be at least 70%, preferably at least 80%, more preferably at least 90%, even more preferably at least 95%, and most preferably at least 98%. A purity within this range is preferred to ensure quality.

As used herein, the terms "about" and "approximately" generally refer to a deviation of ± 10% from the stated value or range, appropriately ± 5%, and particularly ± 1%. Or, for those of ordinary skill in the art, the terms "about" and "approximately" mean within an acceptable standard error range of the mean value.

As used herein, the term "solution" refers to a mixture comprising at least one solvent and at least one compound, wherein the compound is at least partially dissolved in the solvent.

As used herein, the term "solvate" means having a solvent on the surface, in the lattice, or on the surface and in the lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, *t*-butanol, *N,N*-dimethylacetamide, *N,N*-dimethylformamide, formamide, formic acid, heptane, hexane, isopropanol, methanol, methyl ethyl ketone, 1-methyl-2-pyrrolidone, nitromethane, polyethylene glycol, propanol, 2-propanone, pyridine, tetrahydrofuran, toluene, xylene, mixtures thereof, and the like. A specific example of the solvate is a hydrate in which the solvent on the surface, in the lattice or on the surface and in the lattice is water. On the surface, in the lattice or on the the surface and in the lattic of the substance, the hydrate may or may not have any solvent other than water.

Unless otherwise specified, the percentages described throughout this specification are weight/weight (w/w) percentages.

The term "pharmaceutical composition" refers to a mixture of one or more of the compounds described herein, or physiologically/pharmaceutically acceptable salts or prodrugs thereof, and other chemical components, such as physiologically/pharmaceutically acceptable carriers, excipients, diluents, adjuvants, vihicles, and other additional therapeutic agents, such as anti-diabetic agents, antihyperglycemic agents, antiadipositas agents, antihypertensive agents, antiplatelet agents, antiatherosclerotic agents, lipid-lowering agents, etc. The purpose of the pharmaceutical composition is to facilitate administration of a compound to an organism.

The term "syndrome X", also known as conditions, diseases of metabolic syndrome, the disorders are detailed in Johannsson et al., J. Clin. Endocrinol. Metab., 1997; 82, 727-734, which is incorporated herein by reference.

As used herein, the term "treat", "treating" or "treatment" of any disease or disorder refers in some embodiments, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

**Pharmaceutical compositions, formulations, and methods of administration comprising the crystal form or combinations thereof as described in the present invention.**

As described in the present invention, the pharmaceutically acceptable compositions of the present invention further comprise pharmaceutically acceptable adjuvants. These adjuvants, as exemplified in the present invention, include but are not limited to any solvents, solid excipients, adjuvant, binders, disintegrants, or other liquid excipients, dispersant, flavoring agents or suspending agents, surfactants, isotonic agents, thickening agents, emulsifiers, preservatives, solid binders, or lubricants, and the like, as suitable for the specific target dosage form. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, both of which are herein incorporated by reference in their entireties, discloses various carriers used in formulating pharmaceutically acceptable compositions and known techniques for the preparation thereof. Except insofar as any conventional adjuvant incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable adjuvants include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents; releasing agents; coating agents; sweetening; flavoring; perfuming agents; preservatives and antioxidants.

The crystal form or combination or pharmaceutical composition thereof disclosed herein can be administered as the sole pharmaceutical agent or in combination with one or more other additional therapeutic (pharmaceutical) agents where the combination causes no unacceptable adverse effects. This may be of particular relevance for the treatment of diabetes, diabetic complications and other related diseases. Some non-limiting examples of these diseases include diabetes mellitus type I, diabetes type II, diabetic retinopathy, diabetic neuropathy, diabetic nephropathy, insulin resistance, hyperglycemia, hyperinsulinemia, elevated blood levels of fatty acids or glycerol, hyperlipidemia, obesity, hypertriglyceridemia, syndrome X, diabetic complications, atherosclerosis and hypertension. As used herein, the additional therapeutic agents include an anti-diabetic agent other than an SGLT-2 inhibitor, an antihyperglycemic agent, an antiadipositas drug, an antihypertensive agent, an antiplatelet agent, an antiatherosclerotic drug, a lipid-lowering agent, an anti-inflammatory or a combination thereof.

Wherein, the anti-diabetic agents other than an SGLT-2 inhibitor include, but are not limited to, biguanides (e.g., phenformin and metformin), sulfonylureas (e.g., acetohexamide, chlorpropamide, glibenclamide, glipizide, gliclazide, glimepiride, glipentide, gliquidone, tolazamide and tolbutamide, meglitinide), glinides (e.g., repaglinide, nateglinide), $\alpha$-glucoside hydrolase inhibitors (e.g., acarbose), $\alpha$-glucosidase inhibitors (e.g., adiposine, camiglibose, emiglitate, miglitol, voglibose, pradimicin and salbostatin), PPAR agonists (e.g., balaglitazone, ciglitazone, darglitazone, englitazone, isaglitazone, pioglitazone, rosiglitazone and troglitazone), PPAR$\alpha/\gamma$ dual agonists (such as CLX-0940, GW-1536, GW-1929, GW-2433, KRP-297, L-796449, LR-90, MK-0767 and SB-219994), DPP-IV inhibitors (e.g., sitagliptin, vidagliptin, alogliptin, linagliptin and saxagliptin), glucagon-like peptide-1(GLP-1) agonists (e.g., exendin-3 and exendin-4), protein tyrosine phosphatases-1B (PTP-1B) inhibitors (e.g., trodusquemine, hyrtiosal, extract and compounds are disclosed by Zhang, S. et al., Drug Discovery Today, 12(9/10), 373-381, 2007), insulin, insulin analogs, glycogen phosphorylase inhibitors, VPAC2 receptor agonists, glucokinase activators, glycogen phosphorylase inhibitors or glucose-6-phosphatase inhibitors, $\alpha$P2 inhibitors, acetyl-CoA carboxylase-2 (ACC-2) inhibitors, phosphodiesterase (PDE)-10 inhibitors, diacylglycerol acyltransferase (DGAT) 1 or 2 inhibitors, glucose transporter 4 (GLUT4) regulators and a glutamine - fructose - 6 - phosphate amidotransferase (GFAT) inhibitors.

Wherein, the anti-hyperglycemic drugs include, but are not limited to, biguanides (e.g., phenformin and metformin), sulfonylureas (e.g., acetohexamide, chlorpropamide, glibenclamide, glipizide, gliclazide, glimepiride, glipentide, gliquidone, tolazamide and tolbutamide, meglitinide), glinides (e.g., repaglinide, nateglinide), $\alpha$-glucoside hydrolase inhibitors (e.g., acarbose), $\alpha$-glucosidase inhibitors (e.g., adiposine, camiglibose, emiglitate, miglitol, voglibose, pradimicin and salbostatin), PPAR agonists (e.g., balaglitazone, ciglitazone, darglitazone, englitazone, isaglitazone, pioglitazone, rosiglitazone and troglitazone), PPARα/γ dual agonists (such as CLX-0940, GW-1536, GW-1929, GW-2433, KRP-297, L-796449, LR-90, MK-0767 and SB-219994), DPP-IV inhibitors (e.g., sitagliptin, vidagliptin, alogliptin, linagliptin and saxagliptin), glucagon-like peptide-1(GLP-1) agonists (e.g., exendin-3 and exendin-4), protein tyrosine phosphatases-1B (PTP-1B) inhibitors (e.g., trodusquemine, hyrtiosal, extract and compounds are disclosed by Zhang, S. et al., Drug Discovery Today, 12(9/10), 373-381, 2007), insulin, insulin analogs, glycogen phosphorylase inhibitors, VPAC2 receptor agonists, glucokinase activators, glycogen phosphorylase inhibitors or glucose-6-phosphatase inhibitors, αP2 inhibitors, acetyl-CoA carboxylase-2 (ACC-2) inhibitors, phosphodiesterase (PDE)-10 inhibitors, diacylglycerol acyltransferase (DGAT) 1 or 2 inhibitors, glucose transporter 4 (GLUT4) regulators and a glutamine - fructose - 6 - phosphate amidotransferase (GFAT) inhibitors.

Wherein, the lipid-lowering drugs described in the present invention include, but are not limited to, MTP inhibitors, HMG CoA reductase inhibitors (hydroxymethylglutaryl-CoA reductase inhibitors), squalene synthase inhibitors, fibrates (fibric acid derivatives), ACAT inhibitors (acyl-coenzyme A cholesterol acyltransferase inhibitors), lipoxygenase inhibitors, cholesterol absorption inhibitors, ileal sodium/bile acid co-transporter inhibitors, up-regulators of LDL receptor activity, bile acid sequestrants, or nicotinic acid derivatives. In some embodiments, the lipid-lowering drug is selected from pravastatin, simvastatin, atorvastatin, fluvastatin, cerivastatin, atavastatin and rosuvastatin. Wherein, the anti-obesity drugs include CB-1 antagonists (such as rimonabant, taranabant, surinabant, otenabant, SLV319 and AVE1625), gut-selective MTP inhibitors (such as dirlotapide, mitratapide and implitapide), CCKa agonists, 5-$HT_{2c}$ agonists (such as lorcaserin), MCR4 agonists, lipase inhibitors (such as Cetilistat), $PYY_{336}$, opioid antagonist (such as naltrexone), oleoyl-estrone, obinepitide, pramlintide, tesofensine, leptin, liraglutide, bromocriptine, orlistat, exenatide, AOD-9604 and sibutramide.

Wherein, the suitable anti-inflammatory drugs include genital tract/urinary tract infection preventatives and treatments. Exemplary agents include cranberries (Vaccinium macrocarpon) and cranberry derivatives, such as cranberry juice, cranberry extracts or flavonols of cranberries. Moreover, other suitable anti-inflammatory agents include, but are not limited to, aspirin, non-steroidal anti-inflammatory drugs, glucocorticosteroid, sulfasalazine and selective cyclooxygenase-2 inhibitors, etc.

The compositions disclosed herein may be administered orally, parenterally, topically, buccally, or via an implanted reservoir. The term "parenteral" as used herein includes subcutaneous, intravenous, intramuscular, intra-articular, intra-synovial, intrasternal, intrathecal, intraocular, intrahepatic, intralesional and intracranial injection and infusion techniques. The compositions are preferably administered orally, intraperitoneally or intravenously. Sterile injectable forms of the compositions disclosed herein include aqueous and oleaginous suspension. These suspensions may be formulated according to techniques known in the art using suitable dispersing or wetting agents and suspending agents.

The pharmaceutically acceptable compositions of the present invention may be administered orally in any acceptable oral dosage form, including, but not limited to, capsules, tablets, aqueous suspensions or solutions. With regard to the oral use of tablets, carriers generally include lactose and corn starch. Lubricants, such as magnesium stearate, are typically added. For oral administration of capsules, suitable diluents include lactose and dry corn starch. When the oral administration of water as a suspension, the active ingredient is consisted by the emulsifier and suspending agent. If these dosage forms are desired, certain sweeteners, flavoring or coloring agents may also be added.

Liquid dosage forms for oral administration include, but are not limited to, pharmaceutically acceptable emulsions, microemulsions, solutions, suspensions, syrups and elixirs. In addition to the active compound, the liquid dosage form may contain a known general inert diluent such as water or other solvent, solubilizing agent and emulsifier such as ethanol, isopropanol, ethyl carbonate, ethyl acetate, benzyl alcohol, benzoic acid (especially cottonseed, peanut, corn, microbe, olive, castor and sesame oil), glycerol, 2-tetrahydrofuran methanol, polyethylene glycol, diethylene glycol, 1,3-butanediol, dimethylformamide, , Sorbitan fatty acid esters, and the mixtures thereof. In addition to the inert diluent, the oral compositions may also contain adjuvants such as wetting agents, emulsifying or suspending agents, sweetening agents, flavoring agents and fragrances.

Injections, such as sterile injectable or oily suspensions, may be formulated according to techniques known in the art and formulation using suitable dispersing or wetting agents and suspending agents. The sterile injectable agent may be a non-toxic sterile injectable solution, suspension or emulsion made by injection of an acceptable diluent or solvent, for example, a 1,3-butanediol solution. The acceptable vehicles and solvents that include water, Ringer's solution, U. S. P. and isotonic sodium chloride solution. In addition, sterile, non-volatile oil can be conventionally employed as a solvent or suspending medium. For this purpose, any bland non-volatile oil includes synthetic mono- or diglycerides. In addition, fatty acids such as oleic acid can be utilized in the preparation of injectable formulations as natural pharmaceutically acceptable oils, including olive oil or castor oil, particularly their polyoxyethylene derivatives. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The injections may be sterile, such as filtered through a bacterial defensive filter, or in the form of a sterile solid composition, the sterilizing agent may be dissolved or dispersed in a sterile water or other sterile injectable medium in. In order to prolong the effect of the compound of the present invention, it is generally necessary to slow down the absorption of the compound by subcutaneous injection or intramuscular injection. This can be achieved by using a liquid suspension to solve the problem of poor water solubility of the crystal or noncrystal material. The absorbance of the compound depends on its dissolution, which in turn depends on the grain size and the crystal shape. In addition, the delayed absorption of the compound injection can be accomplished by dissolving or dispersing the compound in an oil excipient.

The depot form of the injectable is achieved through a microencapsulated matrix composed of biodegradable polymers, such as poly (lactic-co-glycolic acid). The controlled-release ratio of the compound depends on the compound-to-polymer ratio and the properties of the specific polymer. Other biodegradable polymers include poly (ortho esters) and poly (anhydrides). The depot forms of the injectable can also be prepared by entrapping the compound in liposomes or microemulsions that are compatible with body tissues.

Solid dosage forms for oral administration include capsules, tablets, pills, powders and granules. In these dosage forms, the active compound is combined with at least one pharmaceutically acceptable inert excipient or carrier such as sodium citrate or calcium phosphate and/or a) fillers such as starch, lactose, sucrose, glucose, mannitol and silicic acid, b) binders such as carboxymethylcellulose, alginates, gelatin, polyvinylpyrrolidone, sucrose and acacia, c) humectants such as glycerol, d) disintegrating agents such as agar, calcium carbonate, potato starch or tapioca starch, alginic acid, certain silicates and sodium carbonate, e) blocker solutions such as paraffin, f) absorption enhancers such as quaternary amines, g) wetting agents such as cetyl alcohol and glyceryl monostearate, h) absorbents such as kaolin and bentonite, i) lubricants such as talc, calcium stearate, magnesium stearate, solid polyethylene glycol, sodium lauryl sulfate, and mixtures thereof. In the case of capsules, tablets, and pills, the dosage forms may incorporate buffering agents.

Solid dosage forms can be prepared by coating or capsules, such as enteric coatings, controlled release coatings and other well known pharmaceutical formulation methods to produce tablets, lozenges, capsules, pills, and granules. In these solid dosage forms, the active compound may be mixed with at least one inert diluent, such as sucrose, lactose or starch. Such dosage forms, as a general application, may also include addition substances other than inert diluents, such as tabletting lubricants and other tablet auxiliaries, such as magnesium stearate and microcrystalline cellulose. They may optionally contain emulsifying agents and may also have properties of the composition to optionally release the active ingredient in a delayed manner, or preferably, release it in a specific part of the intestine. Examples of embedding compositions that can be used include polymeric substances and waxes. As for capsules, tablets and pills, these dosage forms may contain buffering agents. Solid compositions of analogous types may also serve as fillers in soft and hard gelatin capsules, utilizing excipients such as lactose or milk sugar and high molecular weight polyethylene glycol.

The compounds or compositions thereof of the present invention are preferably formulated in dosage form according to the formulation to reduce the dose and dose uniformity. The term "dosage unit type" herein refers to a physically dispersed unit in which a patient obtains the appropriate treatment. However, it should be understood that the daily general use of the compounds or compositions of the present invention will be determined by the attending physician on the basis of a reliable medical range judgment. The specific effective dose level for any particular patient or organism will depend on a number of factors including the severity of the disease and condition being treated, the activity of the particular compound, the particular composition used, the age, weight, health, sex and eating habits of the patient, time of administration, route of administration and excretion rate of the particular compound used, duration of treatment, drug use in combination or in combination with a specific compound, and other well known factors in the field of pharmacy.

The "effective amount", "therapeutically effective amount" or "effective dose" of the crystalline compound or pharmaceutically acceptable composition is an amount that is effective in treating or lessening the severity of one or more of the aforementioned disorders. The crystalline compounds and pharmaceutically acceptable compositions are effective administered in a fairly wide dose range. For example, the daily dose is from about 0.1 mg to 1000 mg per person, they can be administered in a single dose or in several divided doses a day. The crystalline compounds and compositions, according to the method disclosed herein, may be administered using any amount and any route of administration which is effective for treating or lessening the severity of the disorder or disease. The exact amount required will vary from subject to subject, depending on the species, age, and general condition of the subject, the severity of the infection, the particular agent, its mode of administration, and the like. A compound or pharmaceutical composition can also be administered with one or more other therapeutic agents as discussed above.

## USE OF THE CRYSTAL FORMS AND PHARMACEUTICAL COMPOSITIONS OF THE PRESENT INVENTION

The amount of the crystalline compound or the crystalline compound in the pharmaceutical compositions disclosed herein is an effective and detectable amount for inhibiting sodium-dependent glucose transporters (SGLTs) activity, especially SGLT-1 activity. SGLT-1 is responsible for reabsorption of D-glucose from kidney spherule filtrate, which inhibits glucose reabsorption in blood vessel and this is beneficial to reduce glucose concentrations in blood. Hence, the compound of the invention would be used for preventing and treating diabetes and related diseases or improving

symptoms of these diseases.

The crystalline compounds disclosed herein would be useful for, but are not limited to, preventing or treating diabetes or related diseases, or lessening diabetes or related diseases, or delaying the progression or onset of diabetes or related diseases or increasing HDL levels in a patient by administering to the patient a compound or a composition disclosed herein in an effective amount. Such diseases include, but are not limited to, diabetes, especially type II diabetes, and insulin resistance, hyperglycemia, hyperinsulinemia, elevated blood levels of fatty acids or glycerol in the blood, hyperlipidemia such as hypertriglyceridemia, obesity, hypertriglyceridemia, syndrome X, diabetic complications such as diabetic retinopathy, diabetic neuropathy, diabetic nephropathy, atherosclerosis and hypertension.

Moreover, the crystalline compounds or pharmaceutical compositions disclosed herein also suit for preventing or treating the damage of diabetes in later stages, such as kidney disease, retinopathy, neuropathy, myocardial infarction, peripheral arterial disease, thrombosis, arteriosclerosis, inflammation, immunological diseases, autoimmune diseases such as AIDS, asthma, osteoporosis, cancer, psoriasis, Alzheimer's disease, schizophrenia and infectious diseases.

Besides being useful for human treatment, these crystalline compounds are also useful for veterinary treatment of animals such as companion animals, exotic animals and farm animals, including mammals, rodents, and the like. In other embodiments, the animals disclosed herein include horses, dogs, and cats. As used herein, the compounds disclosed herein include the pharmaceutically acceptable derivatives thereof.

## Description of the drawings

Figure 1 shows the X-ray powder diffraction (XRPD) pattern of crystal form I of the compound represented by formula (I).

Figure 2 shows the differential scanning calorimetry (DSC) curve of crystal form I of the compound represented by formula (I).

Figure 3 shows the thermogravimetric analysis (TGA) profile of crystal form I of the compound represented by formula (I).

Figure 4 shows the X-ray powder diffraction (XRPD) pattern of crystal form II of the compound represented by formula (I).

Figure 5 shows the variable-temperature X-ray powder diffraction (XRPD) patterns of crystal form I of the compound represented by formula (I).

Figure 6 shows the X-ray powder diffraction (XRPD) pattern of the amorphous form of the compound represented by formula (I).

Figure 7 shows the thermogravimetric analysis (TGA) profile of the amorphous form of the compound represented by formula (I).

Figure 8 shows the dynamic vapor sorption (DVS) isotherm of crystal form I of the compound represented by formula (I).

Figure 9 shows the dynamic vapor sorption (DVS) isotherm of the amorphous form of the compound represented by formula (I).

Figure 10 shows the X-ray powder diffraction (XRPD) pattern of the cocrystal form B of the compound represented by formula (I) with L-proline.

## General Preparation and Identification Methods

The invention will now be further described by way of example without limiting the invention to the scope of the invention.

Skilled in the art can learn from this article to properly improve the experimental parameters to implement the preparation method. Of particular note is that all similar substitutions and modifications to the skilled person is obvious, and they are deemed to be included within the scope in the present invention. Related person can clearly realize and apply the techniques disclosed herein by making some changes, appropriate alterations or combinations to the methods without departing from spirit, principles and scope of the present disclosure.

## General Preparation Method

The structures of the compounds were identified by nuclear magnetic resonance (e.g., $^{1}H$-NMR and $^{13}C$-NMR). $^{1}H$-NMR and $^{13}C$-NMR chemical shifts ($\delta$) were recorded as parts per million (ppm). The $^{1}H$-NMR and $^{13}C$-NMR were performed on a Bruker Ultrashield-400 spectrometer and Bruker Avance III HD 600 spectrometer. The appropriate solvent was deuterated-chloroform ($CDCl_3$), deuterated-methanol ($CD_3OD$) or deuterated-dimethyl sulfoxide (DMSO-$d_6$). TMS

(0 ppm) or chloroform (7.25 ppm) is used as the reference standard. When peak multiplicities were reported, the following abbreviations were used: s (singlet), d (doublet), t (triplet), m (multiplet), br (broadened), dd (doublet of doublets), and dt (doublet of triplets), coupling constant J, when given, was reported in Hertz (Hz).

MS spectra were determined on Agilen-6120 Quadrupole LC/MS mass spectrometer.

The thin-layer silica gel used was Yantai Huanghai HSGF254 silica gel plate.

The silica gel used in column chromatography generally was Qingdao Ocean Chemical Factory 300 to 400 mesh silica gel.

The staring materials of the present invention were known or purchased from Shanghai Accela Company, Energy Company, J&K, Chengdu Aiertai Company, Alfa Company and the like, or they could be prepared by the conventional synthesis methods in the prior art.

Unless otherwise stated, the reactions disclosed herein were carried out in a nitrogen atmosphere.

The term "nitrogen atmosphere" refers to such an atmosphere that a reaction flask was equipped with a balloon or a stainless steel autoclave filled with about 1 L nitrogen.

The term "hydrogen atmosphere" refers to such an atmosphere that a reaction flask was equipped with a balloon or a stainless steel autoclave filled with about 1 L hydrogen.

Unless otherwise stated, the solution used in the examples disclosed herein was an aqueous solution.

In the absence of specific instructions in the examples, the reaction temperature is at room temperature, which is 20°C to 30°C unless otherwise specified.

The reaction temperature and drying temperature in the examples are the temperatures displayed by the monitoring instruments, with an allowable error of ± 5°C.

Crystal forms can be prepared by various methods, including but not limited to crystallization or recrystallization from suitable solvent mixtures, sublimation, transformation from another solid phase, crystallization from supercritical fluid liquids, and spraying. Techniques for crystallization or recrystallization of crystal forms from solvent mixtures include, but are not limited to, solvent evaporation, lowering the temperature of the solvent mixture, crystal seeding of supersaturated solvent mixtures of the compound and/or its salts (crystal seeding), freeze drying of the solvent mixture, and adding antisolvent to the solvent mixture. High-yield crystallization techniques, including polycrystals, can be used to prepare crystal forms.

The crystal forms of drugs (including polymorphs), preparation methods, and characterization of drug crystal forms are discussed in Solid-State Chemistry of Drugs, S. R. Byrn, R. R. Pfeiffer, and J. G. Stowell, 2nd edition, SSCI, West Lafayette, Indiana (1999).

In the crystallization technology utilizing solvents, the solvent is generally selected based on one or more factors, including but not limited to the solubility of the compound, the crystallization technique used, and the vapor pressure of the solvent. A combination of solvents can be used, for example, a compound can be solubilized in the first solvent to obtain a solution, then an antisolvent is added to reduce the solubility of the compound in the solution, and precipitate crystal-forming substances. The antisolvent is a solvent in which the compound has low solubility.

Seed crystals can be added to any crystallization mixture to promote crystallization. Seeding can be used to control the growth of specific polymorphs and/or to control the particle size distribution of the crystalline product. Therefore, the calculation of the required amount of seed crystals depends on the size of the available seed crystals and the desired average size of the product particles, as described in "Programmed Cooling Batch Crystallizers," J. W. Mullin and J. Nyvlt, Chemical Engineering Science, 1971, 26, 369-377. Generally, small-sized seed crystals are needed to effectively control crystal growth in the material. Small-sized seed crystals can be produced by sieving, grinding, or micronization of large crystals, or by microcrystallization of the solution. In crystal grinding or micronization, care should be taken to avoid changing the crystallinity from the desired crystal form (i.e., becoming amorphous or other polymorphs).

The cooled crystalline mixture can be filtered under vacuum, and the separated solid product is washed with a suitable solvent (e.g., a cold recrystallization solvent). After washing, the product can be dried under nitrogen purge to obtain the desired crystal form. The product can be analyzed using appropriate spectroscopic or analytical techniques, including but not limited to, for example, X-ray single crystal diffraction analysis, X-ray powder diffraction (XRPD) analysis, differential scanning calorimetry (DSC), thermogravimetric analysis (TGA), Fourier-transform infrared spectroscopy (FT-IR) analysis, and Raman spectroscopy analysis, to ensure that the crystal form of the compound has been formed.

The following abbreviations are used throughout the specification:

| | |
|---|---|
| g | gram; |
| mL, ml | milliliter; |
| mmol | millimole; |
| V | Volume; |

R.T. Room temperature 25°C ± 5°C;

DMSO dimethylsulfoxide.

This invention is further illustrated by the following examples, which should not be construed as limiting the scope of the disclosure of this invention to the specific steps described therein.

In order to further understand the invention, it is detailed below through examples.

## EXAMPLES

The specific synthesis method of the compound N-(2-dimethylaminoethyl)-1-[4-[4-[[5-[(2S,3R,4S,5S,6R)-6-ethyl-3,4,5-trihydroxy-tetrahydropyran-2-yl]-2-methyl-phenyl]methyl]phenyl]butanoylamino] cyclohexylformamide shown in formula (I) is obtained by referring to Example 1 (i.e., amorphous) in the international application WO2021004498A1.

### Example 1 Crystal Form I of the Compound of Formula (I)

#### 1. Preparation of Crystal Form I

**During the research process, the inventors attempted various methods such as anti-solvent addition, solvent evaporation, and slow cooling to conduct extensive experimental studies to prepare the crystal form of compound (I). However, they found that conventional experimental methods could not obtain the crystal form of the free base, including crystal form I, and it could only be prepared through the following method:**

Step 1: Preparation of L-Proline Cocrystal Form B

Compound of *N*-(2-dimethylaminoethyl)-1-[4-[4-[[5-[(2*S*,3*R*,4*S*,5*S*,6*R*)-6-ethyl-3,4,5-trihydroxy-tetrahydropyran-2-yl]-2-methyl-phenyl]methyl]phenyl]butyramido]cyclohexylformamide (50.00 g, 77.22 mmol) and L-proline (9.20 g, 78.3 mmol) were added to ethanol (150.0 mL) and heated to 60°C to obtain a clear solution, stirred for 30 minutes. The heating was turned off and the reaction was allowed to cool to room temperature naturally, solid precipitated, and stirring was continued overnight. The mixed solvent of ethanol and n-heptane (100.0 mL, $V_{ethanol} : V_{n\text{-}heptane}=1:4$) was added to dilute the reaction mixture. The filter cake was washed with a mixed solvent of ethanol and n-heptane (50.0 mL × 2, $V_{etanol} : V_{n\text{-}heptane}=1:4$), and vacuum dried at 50°C to obtain an off-white solid (40.89 g, 70.70%), which is the compound shown in formula (I) L-proline cocrystal crystal form B.

The off-white solid was characterized by Empyrean X-ray powder diffraction (XRPD) analysis using Cu-Kα radiation, showing characteristic peaks at the following 2θ angles: 4.17°, 5.50°, 8.25°, 10.46°, 10.88°, 11.20°, 11.48°, 12.30°, 12.80°, 13.79°, 14.95°, 15.55°, 16.06°, 16.53°, 16.75°, 17.22°, 17.63°, 18.04°, 18.71°, 19.00°, 19.37°, 19.61°, 19.87°, 20.12°, 20.48°, 21.58°, 22.06°, 22.27°, 22.62°, 23.44°, 23.89°, 24.48°, 24.92°, 25.77°, 26.19°, 26.94°, 30.73°, 31.26°, 31.88°, 35.21°, 39.84°, 41.06° and 42.33°. There is an error tolerance of ± 0.2 °.

Step 2: Preparation of Crystal Form I of the Compound of Formula (I)

The L-proline cocrystal B (5.0 g, 6.77 mmol) obtained in step 1 was added to a mixed solvent of ethanol and water (25.0 mL, $V_{ethanol} : V_{water}=1:1$), heated to 40°C, and a clear liquid was first obtained, and then a large amount of solid was quickly precipitated. The mixture was kept warm and stirred for 1 hour. The heating was stopped, and the reaction was allowed to cool naturally to room temperature. The filter cake was washed with a mixed solvent of ethanol and water (5.00 mL × 2, $V_{ethanol} : V_{water}=1:2$), and vacuum dried at 40°C for 60 hours to obtain an off-white solid (3.47 g, yield 82.16%).

#### 2. Identification of Crystal Form I

(1) Analysis and identification by Empyrean X-ray powder diffraction (XRPD) using Cu-Kα radiation with the following characteristic peaks at an angle of 2θ: 5.02°, 5.72°, 7.16°, 9.06°, 9.99°, 10.27°, 10.55°, 11.36°, 12.67°, 13.47°, 14.29°, 14.96°, 15.55°, 16.16°, 16.84°, 16.96°, 17.68°, 18.19°, 18.36°, 18.52°, 18.94°, 19.78°, 20.64°, 21.10°, 21.44°, 21.85°, 22.22°, 22.48°, 23.08°, 23.69°, 23.80°, 25.02°, 25.43°, 25.70°, 26.38°, 26.74°, 27.18°, 27.68°, 28.62°, 29.53°, 30.01°, 30.52°, 31.08°, 31.53°, 32.01°, 32.60°, 33.34°, 33.92°, 34.28°, 34.57°, 35.31°, 35.73°, 36.25°, 36.90°, 37.37°, 37.86°, 38.43°, 38.92°, 39.46°, 40.00°, 40.94°, 41.54°, 41.92°, 42.39°, 42.99°, 43.48°, 44.08°, 44.72°, 46.41°, 47.24°, 48.64°,

50.41°, 51.52°, 53.19°, 53.99° and 58.28°. There is an error tolerance of ± 0.2 °.

(2) Analysis and identification by TA Q2000 Differential Scanning Calorimetry: the scanning speed is 10 °C/min, and the obtained DSC curve is shown in Figure 2, containing an endothermic peak of 129.31 °C. There is an error tolerance of ± 3 °C.

(3) Analysis and identification by TA Q500 Thermogravimetric analysis: The heating rate is 10 °C/minute, and the resulting TGA curve is shown in Figure 3. The weight loss at 150 °C was 2.106%, with a margin of error of ± 0.1%.

**Example 2 Crystal Form II of the Compound of Formula (I)**

**1. Preparation of Crystal Form II**

The crystal form I of the compound of formula (I) was placed on the sample stage for XRD variable temperature detection, with a heating rate of 10 °C/minute, at 30 °C, 60 °C, 80 °C, 100 °C, 110 °C, and 120 °C (held for 3 minutes each). The system was then cooled down to 30 °C, and XRD detection was performed at each constant temperature stage. During the heating process from 60-120 °C, crystal form II was obtained; however, as the temperature decreased, crystal form II gradually spontaneously transformed back to crystal form I. The resulting variable temperature X-ray powder diffraction (VT-XRPD) pattern is essentially as shown in Figure 5. This indicates that crystal form II can only exist at higher temperatures and cannot stably exist under normal temperature conditions.

**2. Identification of Crystal Form II**

(1) Analysis and identification by Empyrean X-ray powder diffraction (XRPD) using Cu-Kα radiation with the following characteristic peaks at an angle of 2θ: 4.90°, 5.56°, 7.00°, 8.90°, 9.82°, 10.04°, 10.33°, 11.16°, 12.46°, 13.28°, 14.06°, 14.77°, 15.28°, 15.89°, 16.29°, 16.80°, 16.99°, 17.89°, 18.31°, 18.51°, 18.92°, 19.70°, 20.31°, 20.77°, 21.17°, 21.71°, 21.91°, 22.15°, 22.80°, 23.08°, 23.65°, 24.75°, 25.07°, 25.57°, 25.86°, 26.39°, 27.07°, 27.30°, 27.59°, 28.24°, 29.13°, 29.77°, 30.14°, 30.15°, 30.47°, 31.00°, 31.17°, 31.38°, 31.85°, 32.41°, 33.00°, 33.57°, 34.08°, 34.93°, 36.00°, 36.32°, 37.60°, 38.68° and 39.20°. There is an error tolerance of ± 0.2°.

**Example 3 Amorphous form of the compound of Formula (I)**

The amorphous form can be prepared by referring to Example 1 of WO2021004498A1, and can also be obtained through the following preparation method:

Crystal form I of the compound of formula (I) (100 mg, 0.16 mmol)) was added to dichloromethane (3.0 mL), and ultrasonicated until the sample was dissolved. Then, the filtrate was added to a 25 mL single-necked bottle, and the solvent was removed in vacuo at 40 °C on a rotary evaporator, and then vacuum dried at room temperature for 2 hours to obtain a foamy off-white solid (79 mg, yield 79.15%).

**1. Identification of Amorphous form**

(1) Analysis and identification by Empyrean X-ray powder diffraction (XRPD) using Cu-Kα radiation, the resulting X-ray powder diffraction (XRPD) pattern is essentially as shown in Figure 6.

(2) Analysis and identification by TA Q500 Thermogravimetric analysis: The heating rate is 10 °C/minute, and the resulting TGA curve is shown in Figure 7. The weight loss was 3.388%, with a margin of error of ± 0.1%.

**X-ray single crystal diffraction study of crystal form I of the compound of formula (I)**

Data were collected on an Agilent Technologies Gemini A Ultra diffractometer using Cu Kα radiation (λ = 1.5418 Å). The measured intensity data were indexed and processed using the CrysAlis PRO program. The unit cell parameters were determined by preliminary experiments and a data collection strategy was developed based on the unit cell parameters.

The structure solution and refinement were performed using the SHELX-97 (Sheldrick, G. M. SHELXTL-97, Program for Crystal Structure Solution and Refinement; University of Gottingen: Gottingen, Germany, 1997) program, and the solution was performed by the direct method. The derived atomic parameters (coordinates and temperature factors) were corrected by full matrix least squares method. The function minimized in the correction is $\sum_w (|F_o|-|F_c|)^2$. R is defined

as $\sum ||F_o|-|F_c|| / \sum |F_o|$, and $R_w = [\sum_w (|F_o|-|F_c|)^2 / \sum_w |F_o|_2]^{1/2}$, where w is a suitable weighting function based on the error in the observed intensities. Difference maps were checked at all stages of refinement. The positions of hydrogen atoms H1N and H2N were determined from difference Fourier maps, while the positions of other hydrogen atoms were obtained by theoretical calculations. The simulated X-ray powder diffraction pattern was calculated using Mercury software.

A single crystal of appropriate size (crystal form I prepared in Preparation Example 1) was selected for single crystal diffraction analysis. The selected crystals were fixed to a thin glass fiber with a small amount of vaseline and mounted on an Agilent Technologies Gemini A Ultra diffractometer. The diffractometer was measured at a temperature of about 150 K and the unit cell parameters listed in Table 1 were obtained.

Table 1 Cell parameters of crystal form I

| |
|---|
| a = 5.81961(4) Å |
| b = 17.21305(9) A |
| c = 35.51838(19)Å |
| $\alpha$ =90° |
| $\beta$ = 90° |
| $\gamma$ =90° |
| Space group: Orthorhombic, $P\ 2_1\ 2_1\ 2_1$ |
| Z: 4 |
| Volume: 3557.99Å$^3$ |

**The stability test of the crystal form of the present invention**

An appropriate amount of the crystalline form I or amorphous sample of the compound of formula (I) of the present invention was taken and spread flat in a clean petri dish to form a thin layer with a thickness of ≤5 mm, and the test was carried out under the following conditions:

High-temperature test

Take an appropriate amount of the test samples and place them in a flat weighing bottle, spreading into a thin layer ≤5 mm thick. Condition the samples at 60 °C and 75% relative humidity (RH) for 30 days. On the 30th day, collect samples and perform testing according to the stability key investigation items. The purity of the sample is detected by HPLC, as shown in Table 2.

High-humidity test

Take an appropriate amount of the test samples and place them in a flat weighing bottle, spreading into a thin layer ≤5 mm thick. Condition the samples at 25 °C and 92.5% relative humidity (RH) for 30 days. On the 30th day, collect samples and perform testing according to the stability key investigation items.

Light exposure test

Take an appropriate amount of the test samples and place them in a flat weighing bottle, spreading into a thin layer ≤5 mm thick. Leave the bottle uncovered and position it in a UV-equipped light chamber under the following conditions: Illuminance: 4500 ± 500 lx, UV intensity: ≥0.7 W/m$^2$, Temperature: 25 °C, Relative humidity (RH): 60%. Expose the samples for either 15 or 30 days. On day 15 or 30, collect samples and analyze them according to the critical stability parameters.

Under the above experimental conditions, the samples taken were analyzed for impurity content using an HPLC chromatograph through the peak area normalization method. The analysis conditions are as follows:

HPLC was determined on Agilent 1200DAD high pressure liquid chromatography spectrometer (Zorbax Eclipse Plus C18 150×4.6 mm chromatographic column).

HPLC test conditions: Run time: 30 min; Column temperature: 35 °C; Detection wavelength: 210 nm and 225 nm; Mobile phase: Phase C: acetonitrile, Phase D: ultrapure water; Flow rate: 1.0 mL/min;

Gradient elution, with the elution ratio as shown in Table A.

Table A:

| Time | Gradient of Mobile Phase C | Gradient of Mobile Phase D |
|---|---|---|
| 0 min | 20% | 80% |
| 4.0 min | 37% | 63% |
| 12 min | 50% | 50% |
| 20 min | 90% | 10% |
| 30 min | 90% | 10% |

Table 2 Change in Impurity Increase △% Before and After Stability Testing for Crystal Form I and Amorphous

| Crystal Form | High Temperature | High Humidity | Light Exposure |
|---|---|---|---|
| Amorphous | 5.10 (30 days) | 1.29 (30 days) | 5.51 (30 days) |
| Crystal Form I | 0.35 (30 days) | 0 (30 days) | 0.07 (15 days) |
| △% indicates the mass fraction of impurities before and after the stability experiment. | | | |

The test results demonstrated that under high-temperature, high-humidity, and light-exposure conditions, the amorphous form of the compound of Formula (I) in the present invention exhibited a significant increase in impurities, with particularly pronounced growth under high-temperature and light-exposure conditions. In contrast, Form I maintained relatively stable chemical purity under these conditions, showing no notable changes, thereby indicating excellent stability and suitability for pharmaceutical applications.

**Hygroscopicity Test**

Test method: According to the test method specified in the "Guidelines for Drug Hygroscopicity Test" in the 4th part of the "Chinese Pharmacopoeia" (2015 edition): Take a dried glass weighing bottle with a stopper (outer diameter of 50mm, height of 15 mm) and place it in a suitable 25 °C ± 1°C constant temperature dryer (with ammonium chloride or ammonium sulfate saturated solution at the bottom) the day before the test. Use a Mettler XP205DR analytical balance to precisely weigh the empty weighing bottle with the stopper, and record the weight as $m_1$. Take an appropriate amount of sample, spread it evenly in the above weighing bottle, with a sample thickness generally about 1 mm, and weigh precisely, recording the weight as $m_2$. Leave the weighing bottle open, and place it with the lid under the above constant temperature and humidity conditions for 24 hours. Cover the weighing bottle with the lid, weigh precisely, and record the weight as $m_3$, then calculate the percentage of weight gain (%).

$$\text{Percentage Weight Increase (\%)} = \frac{m_3 - m_2}{m_2 - m_1} \times 100\%$$

Take the appropriate amount of the test sample, the hygroscopicity is tested by dynamic moisture adsorption device.

Among them, the hygroscopicity experiment DVS graphs of crystal form I and amorphous form of this invention are basically as shown in Figures 8 and 9, and the specific experimental results are shown in Table 3.

Table 3 Hygroscopicity Tests of the Crystal Form of the Invention

| Test sample | Weight Increase (%) at 80% Relative Humidity | Weight Increase (%) at 95% Relative Humidity |
|---|---|---|
| Crystal Form I | 1.515 | 1.628 |
| Amorphous | 4.544 | 9.790 |

According to the description of hygroscopicity characteristics and the criteria for hygroscopic weight gain (Guidelines for Hygroscopicity Testing of Drugs, General Rule 9103, Chinese Pharmacopoeia 2015 Edition, see Table 4 for details).

Table 4 Description of Hygroscopic Characteristics and Definition of Hygroscopic Weight Gain (25 °C ± 1°C, 80% ± 2% Relative Humidity)

| The hygroscopicity feature | Hygroscopic Weight Gain Rate |
|---|---|
| Deliquescence | Absorb enough water to form a liquid |
| Highly hygroscopicity | Hygroscopic weight gain not less than 15% |
| Hygroscopicity | Hygroscopic weight gain less than 15% but not less than 0.2% |
| Lightly hygroscopicity | Hygroscopic weight gain less than 2% but not less than 0.2% |
| No or almost none hygroscopicity | Hygroscopic weight gain less than 0.2% |

The experimental results indicate that crystal form I of the present invention shows little weight gain and slight hygroscopicity.

**Crystallinity experiment of the compound of formula (I) described in this invention**

1. Suspended Stirring Method

50 mg/portion of the compound of formula (I) was placed in a glass bottle, and a certain amount of solvent was added respectively. The obtained suspension was stirred at room temperature, 50°C and 60°C for a certain period of time, and then the solid was collected by filtration and subjected to X-ray powder diffraction test. The results are shown in the following table. No new crystal form was obtained in all suspension stirring tests.

| Solvent system (v : v) | Temperature/°C | Result |
|---|---|---|
| Water | R.T. | Gel-like substance |
| Methyl tert-Butyl Ether | R.T. | Gel-like substance |
| n-Heptane | R.T. | Low crystallinity |
| Isopropyl acetate | R.T. | Gel-like substance |
| Ethanol/Isopropyl ether 1:10 | R.T. | Gel-like substance |
| Acetone/Isopropyl ether 1:10 | R.T. | Gel-like substance |
| Dichloromethane/Isopropyl ether 1:10 | R.T. | Gel-like substance |
| Ethanol/n-Heptane 1:10 | R.T. | Oily substance |
| Acetone/n-Heptane 1:10 | R.T. | Low crystallinity |
| Dichloromethane/n-Heptane 1:10 | R.T. | Low crystallinity |
| THF/n-Heptane 1:10 | R.T. | Low crystallinity |
| Ethyl formate/n-Heptane 1:1 | R.T. | Low crystallinity |
| Chloroform/n-Heptane 1:10 | R.T. | Low crystallinity |
| n-Heptane | 50 | Low crystallinity |
| n-Heptane/Methyl tert-Butyl Ether 1:1 | 50 | Low crystallinity |
| Isopropyl ether | 60 | Gel-like substance |

2. Antisolvent Addition Method-Binary Solvent System

A certain amount of compound of formula (I) was placed in a glass bottle, a certain amount of good solvent was added, dissolved at a certain temperature, and a certain amount of antisolvent, such as water, n-heptane, methyl tert-butyl ether, etc., was added dropwise. After a certain period of time, the solid was collected by filtration and subjected to X-ray powder diffraction testing. The results are shown in the table below, and no new crystal form was obtained in all experiments.

| Good solvent | Anti-solvent | Temperature/°C | Crystallization method | Result |
|---|---|---|---|---|
| Ethanol | Water | R.T. | Forward dropwise addition | Oily substance |
| Ethanol | Water | R.T. | Reverse dropwise addition | Oily substance |
| Ethanol | Methyl tert-Butyl Ether | R.T. | Forward dropwise addition | Oily substance |
| Ethanol | n-Heptane | R.T. | Forward dropwise addition | Oily substance |
| Ethyl acetate | Methyl tert-Butyl Ether | 55 | Forward dropwise addition | Gel-like substance |
| Ethyl acetate | n-Heptane | 77 | Forward dropwise addition | Low crystallinity |
| DCM | n-Heptane | R.T. | Forward dropwise addition | Oily substance |
| Acetone | n-Heptane | R.T. | Forward dropwise addition | Oily substance |
| Methanol | Methyl tert-Butyl Ether | 50 | Forward dropwise addition | Oily substance |

3. Anti-solvent Addition Method-Ternary Solvent System Crystallization Method

A certain amount of compound of formula (I) was placed in a glass bottle, a certain amount of good solvent was added, heated to a certain temperature and stirred, then a certain amount of hydrotropy was added for dissolution. After clearing, a certain amount of antisolvent was dripped in, then cooled to room temperature or - 20 °C, etc. After a certain period, the solid was collected by filtration and subjected to X-ray powder diffraction testing. The results are shown in the table below, and no new crystal form was obtained in all experiments.

| Good solvent | Co-solvent | Anti-solvent | Heating temperature/°C | Cooling temperature/°C | Result |
|---|---|---|---|---|---|
| Ethyl acetate | Ethanol | n-Heptane | 77 | R.T. | Low crystallinity |
| Ethyl acetate | Acetone | n-Heptane | 55 | R.T. | Low crystallinity |
| Ethyl acetate | Acetonitrile | n-Heptane | 77 | R.T. | Low crystallinity |
| Ethyl formate | Ethanol | n-Heptane | 50 | R.T. | Gel-like substance |
| Ethyl formate | Acetone | n-Heptane | 50 | R.T. | Gel-like substance |
| Ethyl acetate | Tetrahydrofuran | n-Heptane | 60 | -20 | Gel-like substance |
| Ethyl acetate | Chloroform | n-Heptane | 60 | -20 | Low crystallinity |
| Ethyl formate | Acetone | n-Heptane | 50 | -20 | Gel-like substance |
| Ethyl formate | Acetone | Methyl tert-Butyl Ether | 50 | -20 | Oily substance |
| Ethyl formate | Chloroform | n-Heptane | 50 | -20 | Gel-like substance |
| Ethyl formate | Chloroform | Methyl tert-Butyl Ether | 50 | -20 | Oily substance |

4. Cooling Crystallization Method

A certain amount of compound of formula (I) was placed in a glass bottle, a certain amount of good solvent was added, and it was heated to 77 °C to dissolve. After cooling to crystallize, the solid was collected by filtration after a certain

period and subjected to X-ray powder diffraction testing. The results are shown in the table below, and no new crystal form was obtained.

| Good solvent | Heating Temperature/°C | Cooling temperature/°C | Result |
|---|---|---|---|
| Ethyl acetate | 77 | R.T. | Gel-like substance |

5. Melting and Cooling Method

A certain amount of compound of formula (I) was vacuum heated to 130 °C to melt, maintained at this temperature for a certain period, then cooled to room temperature. After a certain period, the solid was collected and subjected to X-ray powder diffraction testing. The results are shown in the table below, and no new crystal form was obtained.

| Condition | Heating Temperature/°C | Cooling temperature/°C | Result |
|---|---|---|---|
| Vacuum | 130 | R.T. | Amorphous |

6. Solvent Evaporation Method

A certain amount of the compound of formula (I) was dissolved in a certain solvent, and the solvent was evaporated at 60 °C in vacuum. After a certain period of time, the solid was collected and subjected to an X-ray powder diffraction test. The results are shown in the following table, and no new crystal form was obtained.

| Solvent | Temperature/ °C | Result |
|---|---|---|
| Ethanol | 60 | Amorphous |
| DCM | 60 | Amorphous |
| Tetrahydrofuran | 60 | Amorphous |

7. Gas-Solid Permeation Method

30 mg/portion of the compound of formula (I) was placed in a 5 mL vial, and about 3 mL of solvent was added to another 30 mL vial. The 5 mL vial was opened and placed in a 30 mL vial. After sealing, the vial was allowed to stand at room temperature for 5 days. The solid was collected and subjected to an X-ray powder diffraction test. The results are shown in the following table. No new crystal form was obtained in the gas-solid permeation test.

| Solvent | Result |
|---|---|
| Ethyl acetate | Low crystallinity |
| i-propanol | Low crystallinity |

8. Polymer Induction Method

A certain amount of compound of formula (I) was heated and dissolved in ethyl acetate, a certain amount of polymer was added, and after maintaining the temperature and stirring for a certain period, it was cooled to room temperature. After a certain period, the solid was collected and subjected to X-ray powder diffraction testing. The results are shown in the table below, and no new crystal form was obtained.

| Solvent | Temperature/ °C | Polymer | Result |
|---|---|---|---|
| Ethyl acetate | 80 | Sodium alginate | Amorphous |
| Ethyl acetate | 80 | Polyvinyl chloride | Amorphous |

The above results indicate that it is difficult to obtain the crystal form of the free base shown in formula (I) through

the aforementioned numerous conventional experiments.

Reference throughout this specification to "an embodiment", "some embodiments", "one embodiment", "another example", "an example", "a specific examples", or "some examples" means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example", "in an example", "in a specific examples", or "in some examples", in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure. All publications or patents cited in the present invention are incorporated by reference into the present invention.

## Claims

1. A crystal form of the compound of formula (I), wherein the crystal form is crystal form I,

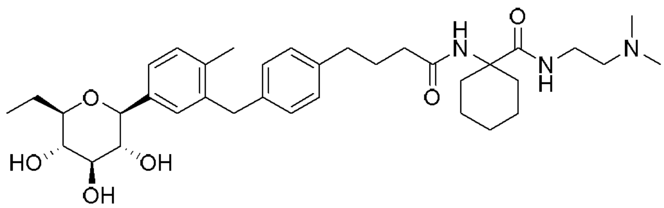

(I).

2. The crystal form according to claim 1, which is **characterized by** an X-ray powder diffraction pattern comprising peaks expressed as 20 at 5.02° ± 0.2°, 9.99° ± 0.2°, 12.67° ± 0.2°, 14.96° ± 0.2°, 15.55° ± 0.2°, 20.64° ± 0.2°.

3. The crystal form according to claim 1 or 2, which is **characterized by** an X-ray powder diffraction pattern comprising peaks expressed as 20 at 5.02° ± 0.2°, 9.06° ± 0.2°, 9.99° ± 0.2°, 12.67° ± 0.2°, 13.47° ± 0.2°, 14.96° ± 0.2°, 15.55° ± 0.2°, 16.16° ± 0.2°, 18.19° ± 0.2°, 20.64° ± 0.2°, 25.02° ± 0.2°.

4. The crystal form according to any one of claims 1-3, which is **characterized by** an X-ray powder diffraction pattern comprising peaks expressed as 20 at 5.02° ± 0.2°, 5.72° ± 0.2°, 7.16° ± 0.2°, 9.06° ± 0.2°, 9.99° ± 0.2°, 10.27° ± 0.2°, 10.55° ± 0.2°, 11.36° ± 0.2°, 12.67° ± 0.2°, 13.47° ± 0.2°, 14.29° ± 0.2°, 14.96° ± 0.2°, 15.55° ± 0.2°, 16.16° ± 0.2°, 16.84° ± 0.2°, 16.96° ± 0.2°, 17.68° ± 0.2°, 18.19° ± 0.2°, 18.36° ± 0.2°, 18.52° ± 0.2°, 18.94° ± 0.2°, 19.78° ± 0.2°, 20.64° ± 0.2°, 21.10° ± 0.2°, 21.44° ± 0.2°, 21.85° ± 0.2°, 22.22° ± 0.2°, 22.48° ± 0.2°, 23.08° ± 0.2°, 23.69° ± 0.2°, 23.80° ± 0.2°, 25.02° ± 0.2°, 25.43° ± 0.2°, 25.70° ± 0.2°, 26.38° ± 0.2°, 26.74° ± 0.2°, 27.18° ± 0.2°, 27.68° ± 0.2°, 28.62° ± 0.2°, 29.53° ± 0.2°, 30.01° ± 0.2°, 30.52° ± 0.2°, 31.08° ± 0.2°, 31.53° ± 0.2°, 32.01° ± 0.2°, 32.60° ± 0.2°, 33.34° ± 0.2°, 33.92° ± 0.2°, 34.28° ± 0.2°, 34.57° ± 0.2°, 35.31° ± 0.2°, 35.73° ± 0.2°, 36.25° ± 0.2°, 36.90° ± 0.2°, 37.37° ± 0.2°, 37.86° ± 0.2°, 38.43° ± 0.2°, 38.92° ± 0.2°, 39.46° ± 0.2°, 40.00° ± 0.2°, 40.94° ± 0.2°, 41.54° ± 0.2°, 41.92° ± 0.2°, 42.39° ± 0.2°, 42.99° ± 0.2°, 43.48° ± 0.2°, 44.08° ± 0.2°, 44.72° ± 0.2°, 46.41° ± 0.2°, 47.24° ± 0.2°, 48.64° ± 0.2°, 50.41° ± 0.2°, 51.52° ± 0.2°, 53.19° ± 0.2°, 53.99° ± 0.2°, 58.28° ± 0.2°.

5. The crystal form according to any one of claims 1-4, which is **characterized by** an X-ray powder diffraction pattern substantially as shown in Figure 1.

6. The crystal form according to any one of claims 1-5, which is **characterized by** a differential scanning calorimetry thermogram comprising an endothermic peak at 129.31 °C ± 3 °C.

7. The crystal form according to any one of claims 1-6, which is **characterized by** a differential scanning calorimetry substantially as described in Figure 2.

8. The crystal form according to any one of claims 1-7, which is **characterized by** the following unit cell parameters of

crystal form I:

unit cell dimensions: a = 5.81961 Å, b = 17.21305 Å, c = 35.51838 Å, $\alpha$ = 90°, $\beta$ = 90°, $\gamma$ = 90°;
Space group: Orthorhombic, $P2_12_12_1$;
Unit cell volume: 3557.99 $Å^3$;
The number of asymmetric units Z in the unit cell is: 4.

**9.** A method for preparing crystal form I of the compound of formula (I) according to any one of claims 1-7, which is **characterized by**: the compound shown in formula (I) and L-proline in an organic solvent, heating, stirring, cooling, precipitating crystals, and filtering to obtain a cocrystal of the compound shown in formula (I) and L-proline. Then, a mixed solvent is added, heated, and crystals are precipitated, filtered, and the filter cake is dried to obtain crystal form I of the compound shown in formula (I).

**10.** The method according to claim 9, wherein the organic solvent is selected from methanol, ethanol, isopropanol, or n-propanol, and the mixed solvent is selected from a mixed solvent of methanol and water, ethanol and water, isopropanol and water, or n-propanol and water.

**11.** A pharmaceutical composition comprising the crystal form according to any one of claims 1-9, and optionally further comprising a pharmaceutically acceptable carrier, excipient, adjuvant, vehicle, or a combination thereof.

**12.** The pharmaceutical composition according to claim 11, further comprising one or more additional therapeutic agent, wherein the additional therapeutic agent is selected from an anti-diabetic agent, an antihyperglycemic agent, an antiobesity agent, an antihypertensive agent, an appetite suppressant, a lipid-lowering agent or a combination thereof;
wherein the antidiabetic drugs and antihyperglycemic drugs of the present invention are independently selected from SGLT2 inhibitors, biguanides, sulfonylureas, glucosidase inhibitors, PPAR agonists, $\alpha$P2 inhibitors, PPAR$\alpha/\gamma$ dual activators, dipeptidyl peptidase IV inhibitors, glinides, insulin, glucagon-like peptide-1 inhibitors, PTP1B inhibitors, glycogen phosphorylase inhibitors, glucose-6-phosphatase inhibitors, or combinations thereof. The anti-obesity drugs are selected from central anti-obesity drugs, MCH receptor antagonists, neuropeptide Y receptor antagonists, cannabinoid receptor antagonists, brain-gut peptide antagonists, lipase inhibitors, $\beta$3 agonists, 11$\beta$-HSD1 inhibitors, DGAT-1 inhibitors, peptide appetite suppressants, cholecystokinin agonists, appetite suppressants, or combinations thereof. The lipid-lowering drugs are selected from MTP inhibitors, HMG-CoA reductase inhibitors, squalene synthase inhibitors, betaine lipid-lowering drugs, ACAT inhibitors, lipoxygenase inhibitors, cholesterol absorption inhibitors, ileal sodium/bile acid co-transporter inhibitors, LDL receptor activity up regulators, niacin lipid-lowering drugs, bile acid chelates, or combinations thereof. The lipid-lowering drugs are selected from pravastatin, simvastatin, atorvastatin, fluvastatin, cerivastatin, pitavastatin, rosuvastatin, or combinations thereof.

**13.** Use of the crystal form according to any one of claims 1 to 9 or the pharmaceutical composition according to any one of claims 11 to 12 in the manufacture of a medicament, wherein the medicament is used for inhibiting SGLT1; or for improving the intestinal environment; or for preventing or treating a disease, lessening a disease symptoms or delaying the progression or onset of a disease, wherein the disease is diabetes, diabetic complications, insulin resistance, hyperglycemia, hyperinsulinemia, hyperlipidemia, obesity, syndrome X, atherosclerosis, cardiovascular diseases, congestive heart failure, hypomagnesemia, hyponatremia, renal failure, disorders related to hemoconcentration, constipation, or hypertension;
wherein the diabetic complications are diabetic retinopathy, diabetic neuropathy, or diabetic nephropathy; and the hyperlipidemia is hypertriglyceridemia.

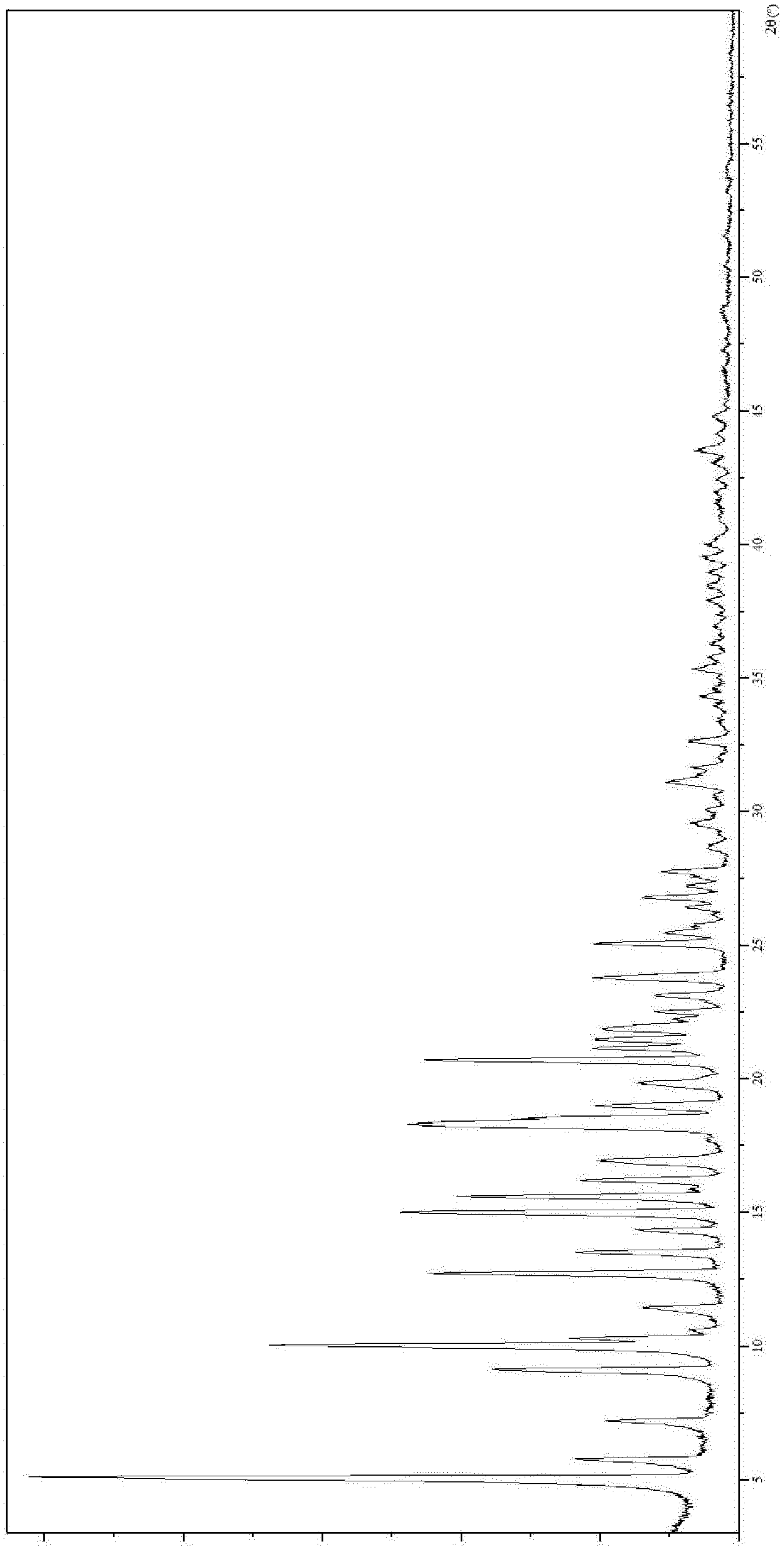
Intensity (counts)
2θ(°)
0
1000
2000
3000
4000
5000
5
10
15
20
25
30
35
40
45
50
55

Figure 1

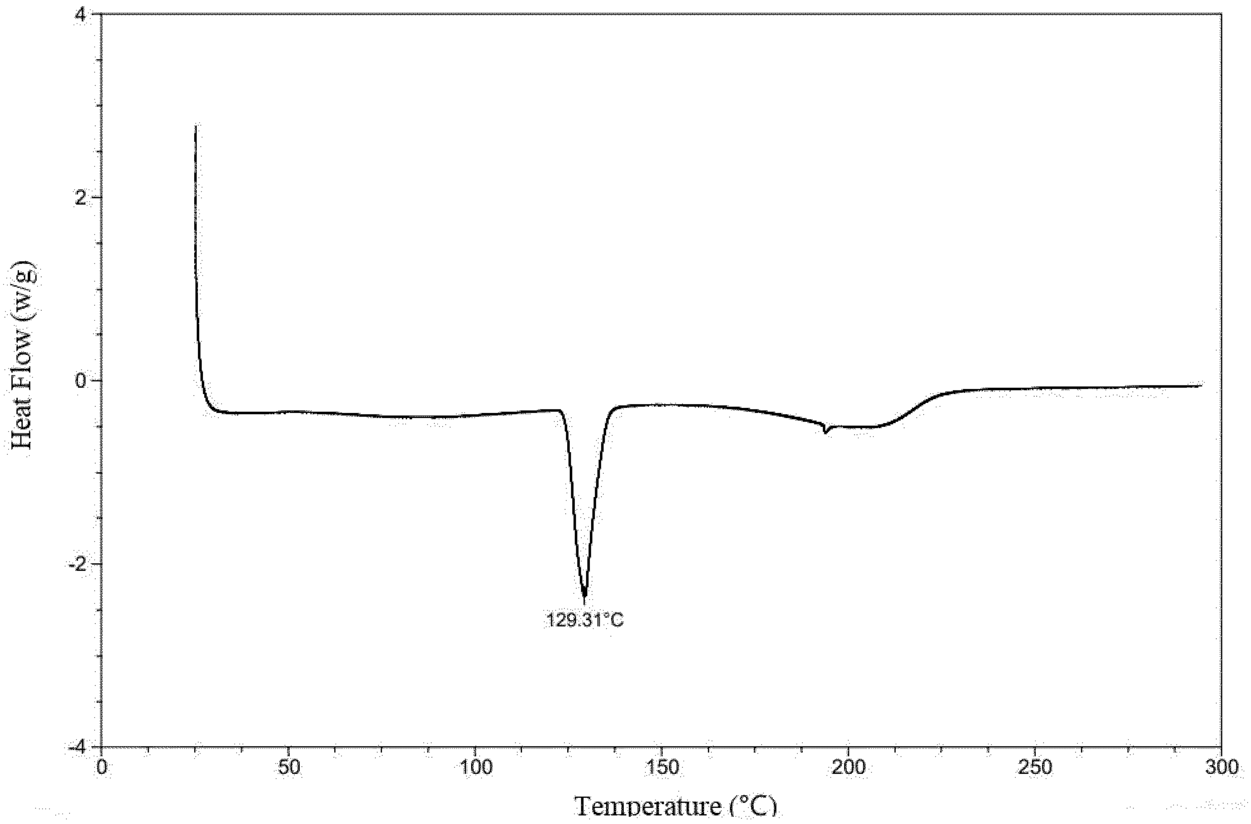


Figure 2

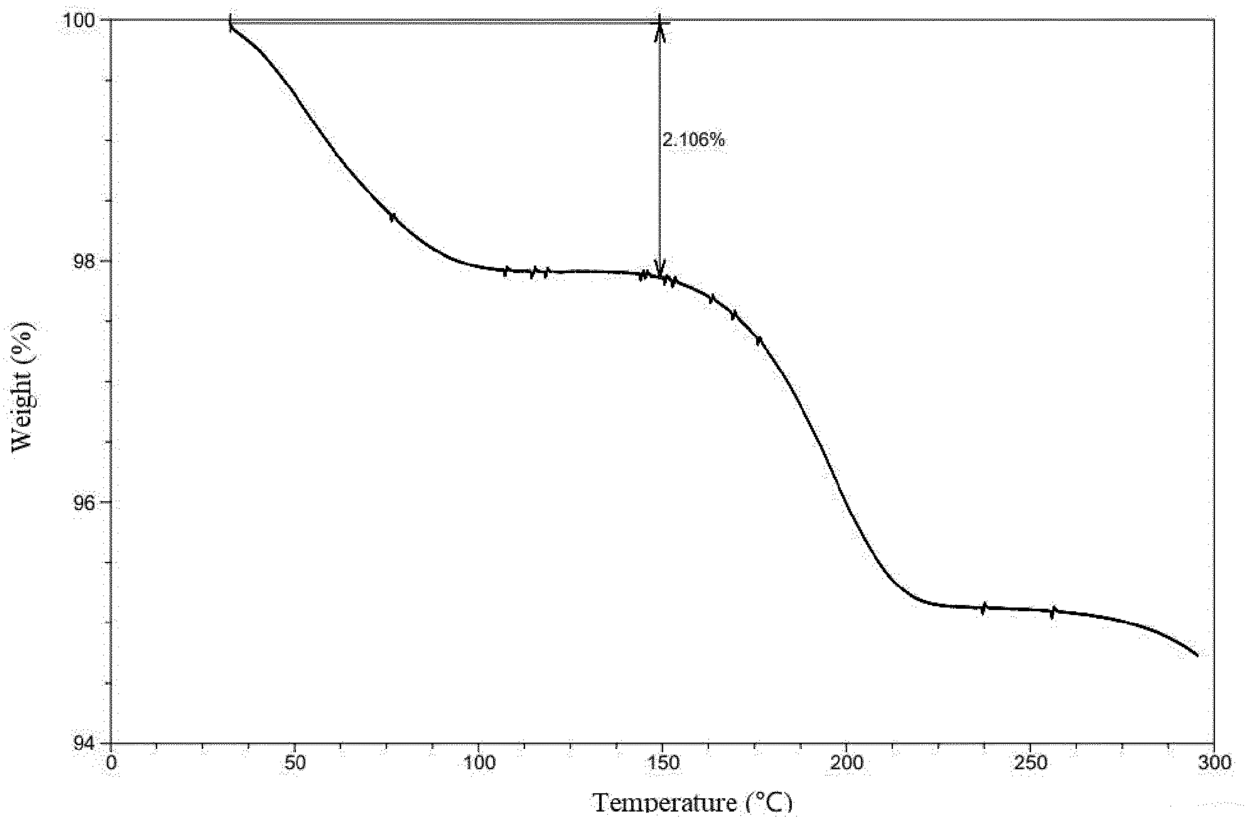


Figure 3

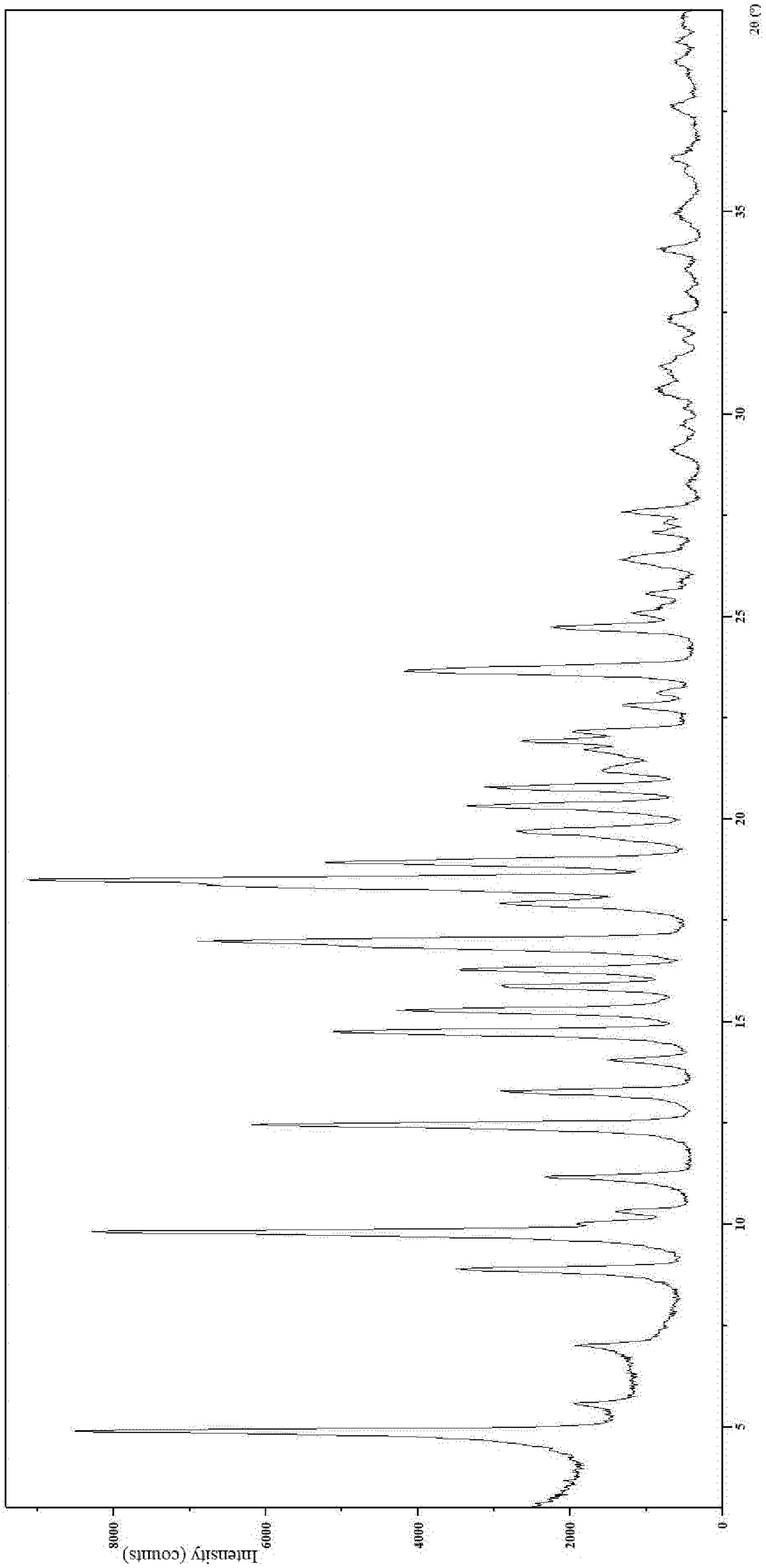


Figure 4

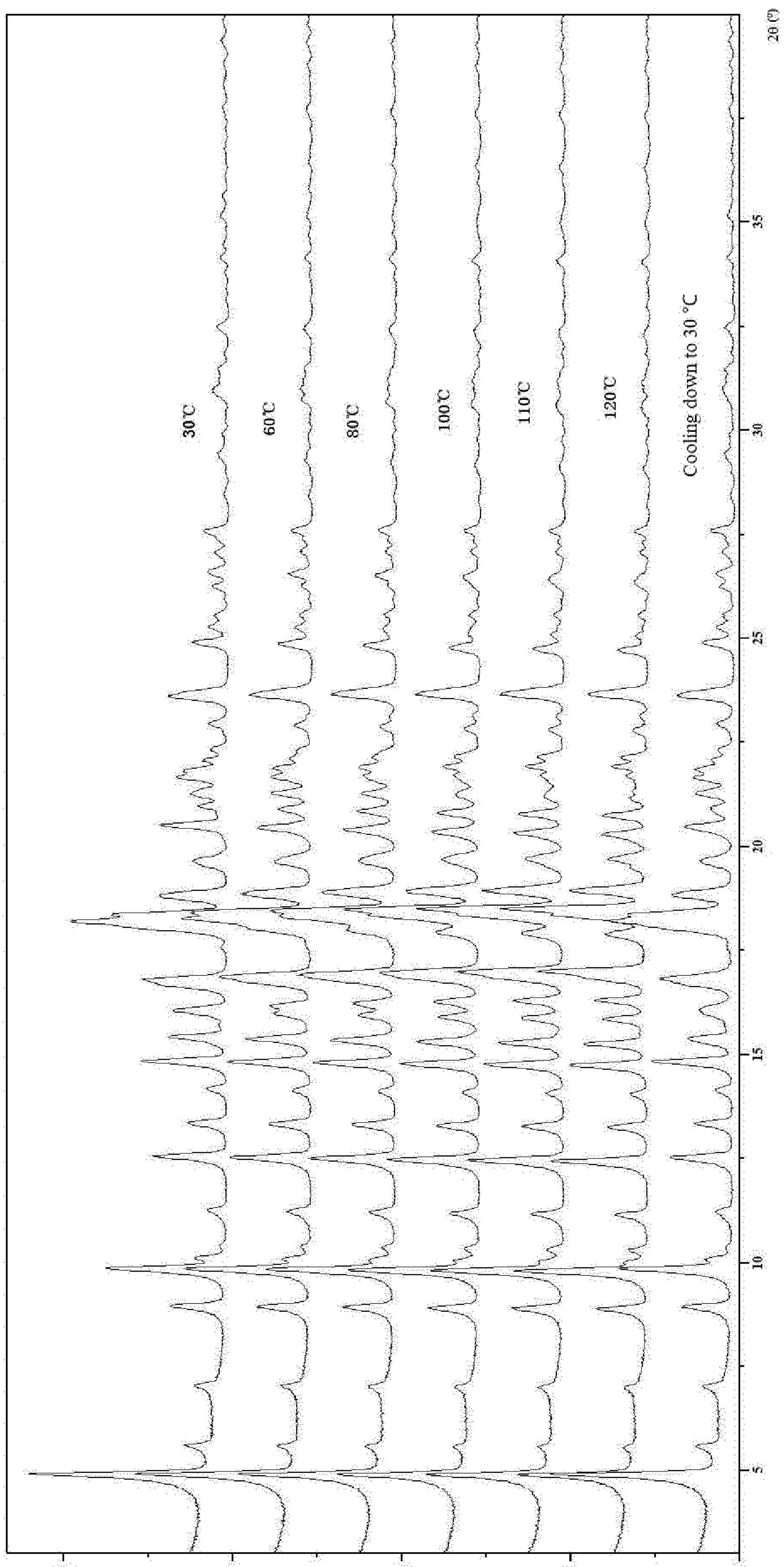
30℃
60℃
80℃
100℃
110℃
120℃
Cooling down to 30 °C
Intensity (counts)
2θ (°)
0
10000
20000
30000
40000
5
10
15
20
25
30
35

Figure 5

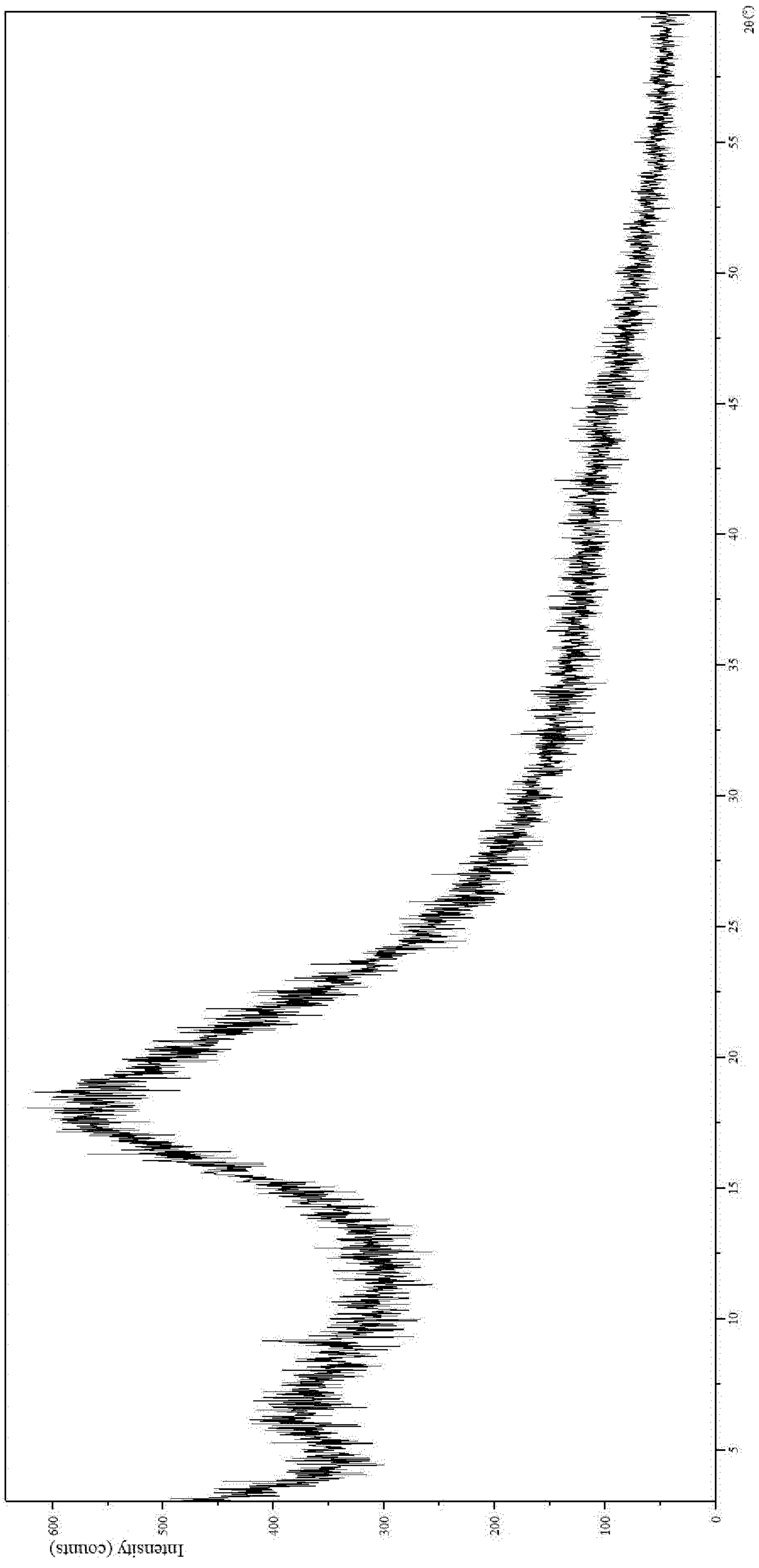
Intensity (counts)
2θ(°)
0
100
200
300
400
500
600
5
10
15
20
25
30
35
40
45
50
55

Figure 6

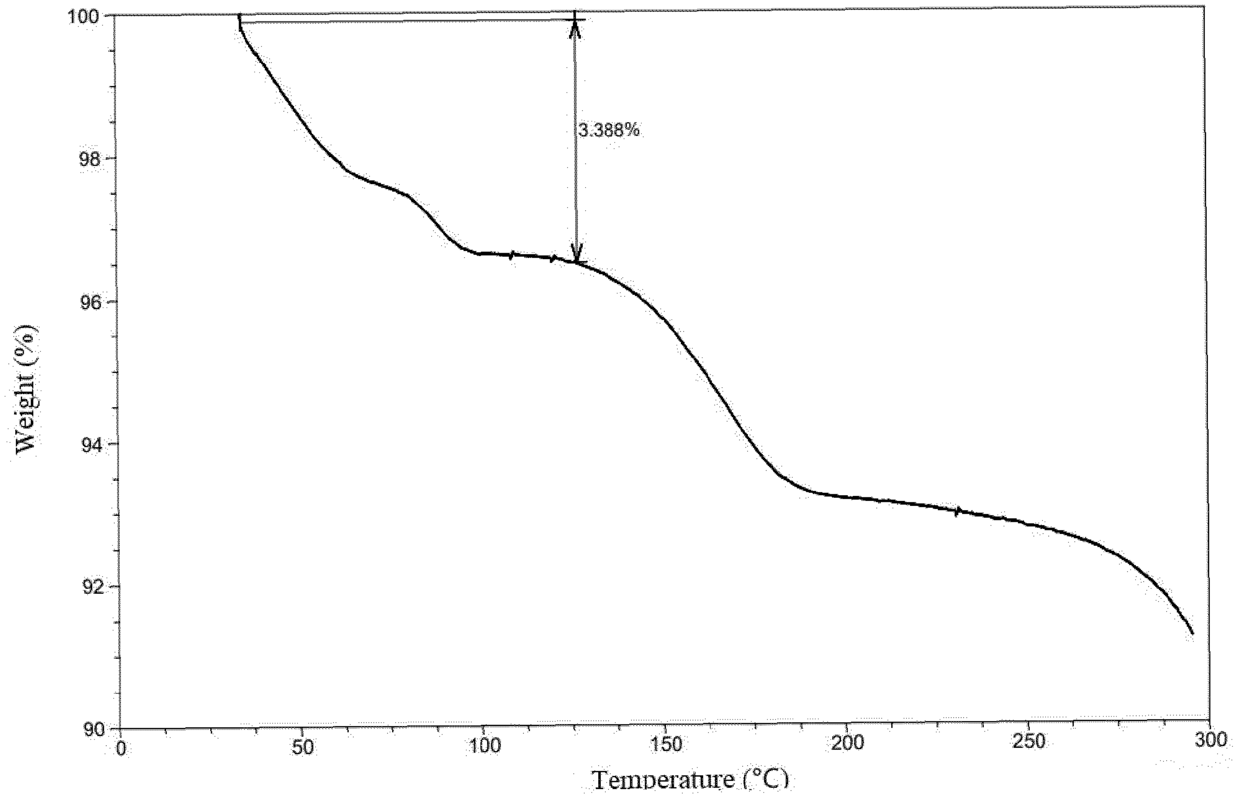


Figure 7

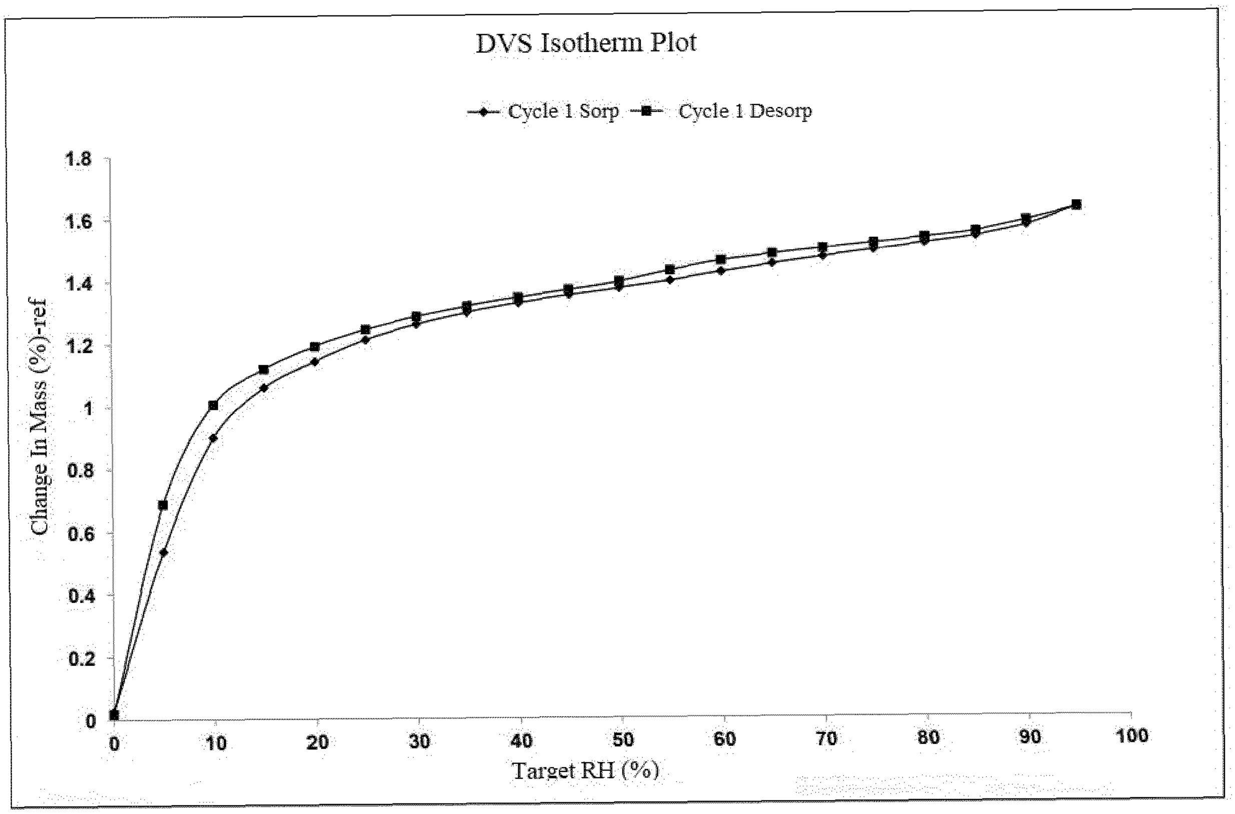


Figure 8

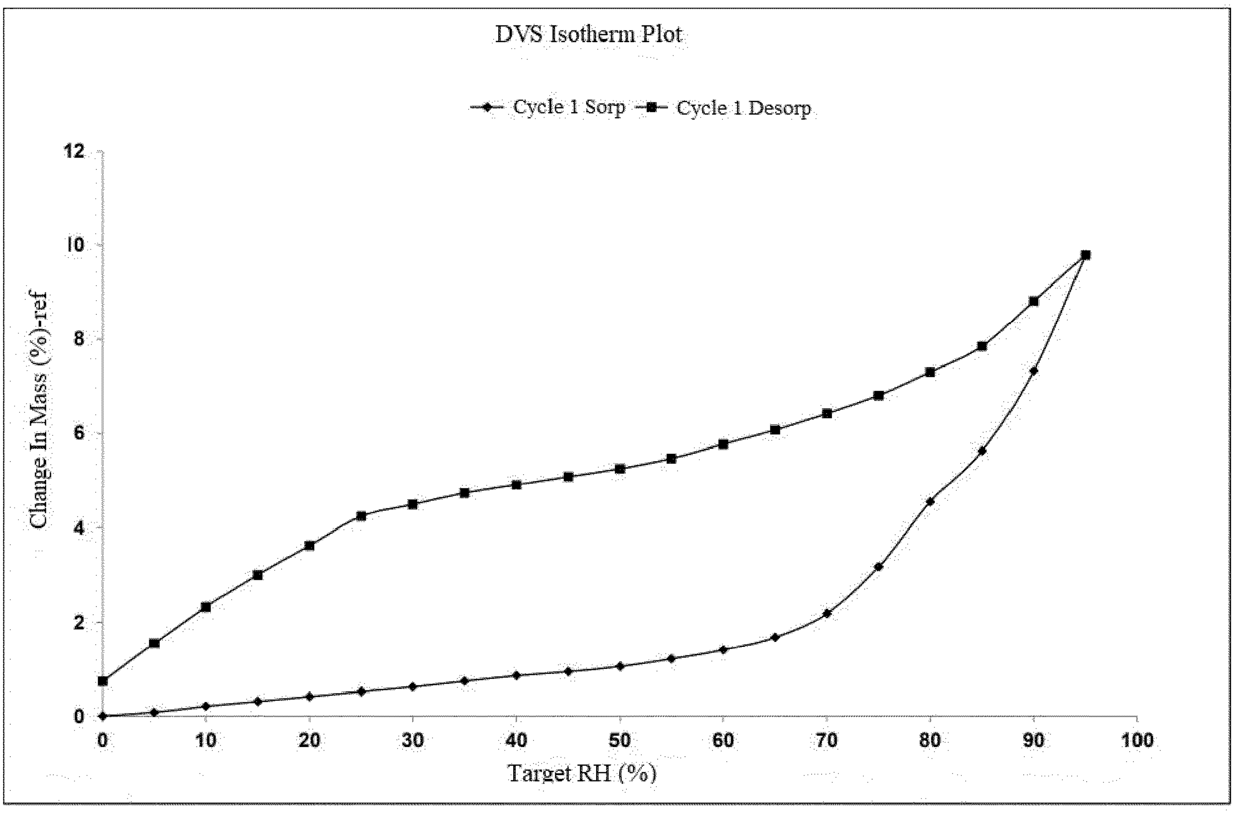
DVS Isotherm Plot
Cycle 1 Sorp
Cycle 1 Desorp
Change In Mass (%)-ref
12
10
8
6
4
2
0
0
10
20
30
40
50
60
70
80
90
100
Target RH (%)

Figure 9

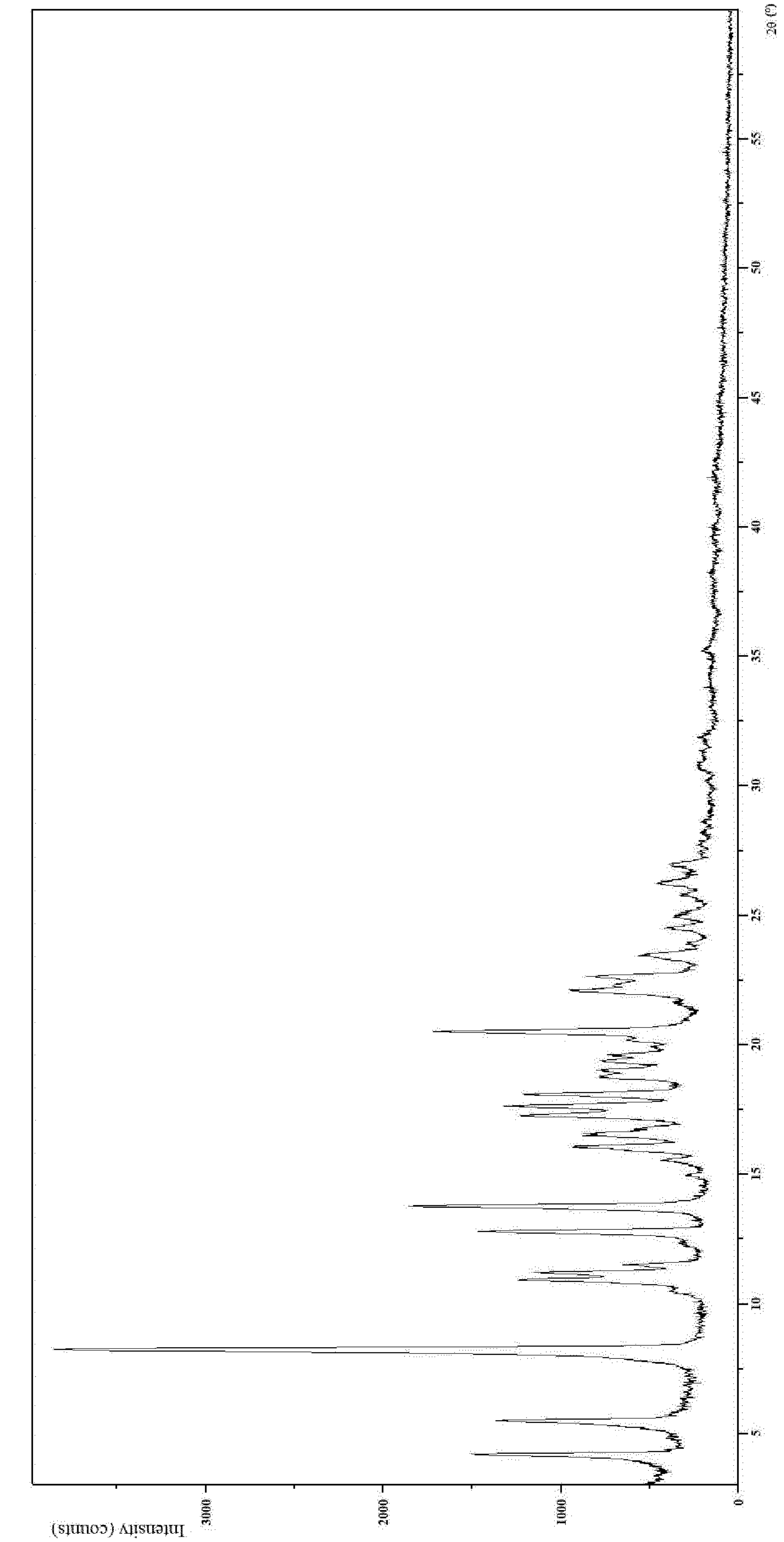
Intensity (counts)
3000
2000
1000
0
5
10
15
20
25
30
35
40
45
50
55
2θ (°)

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021004498 A1

### Non-patent literature cited in the description


- Handbook of Chemistry and Physics. 1994
- Organic Chemistry. Thomas Sorrell, 1999
- **MICHAEL B. SMITH** ; **JERRY MARCH**. March's Advanced Organic Chemistry. John Wiley & Sons, 2007
- Chinese Pharmacopoeia. 2015
- **JOHANNSSON et al.** *J. Clin. Endocrinol. Metab.*, 1997, vol. 82, 727-734
- Remington: The Science and Practice of Pharmacy. Lippincott Williams& Wilkins, 2005
- Encyclopedia of Pharmaceutical Technology. Marcel Dekker, 1988
- **ZHANG, S. et al.** *Drug Discovery Today*, 2007, vol. 12 (9/10), 373-381
- **S. R. BYRN** ; **R. R. PFEIFFER** ; **J. G. STOWELL**. Solid-State Chemistry of Drugs. West Lafayette, 1999
- **J. W. MULLIN** ; **J. NYVLT**. Programmed Cooling Batch Crystallizers. *Chemical Engineering Science*, 1971, vol. 26, 369-377
- **SHELDRICK, G. M.** *SHELXTL-97, Program for Crystal Structure Solution and Refinement*, 1997
- Guidelines for Drug Hygroscopicity Test. Chinese Pharmacopoeia. 2015
- Guidelines for Hygroscopicity Testing of Drugs. Chinese Pharmacopoeia. 2015